# EUROPEAN PATENT SPECIFICATION

(11) **EP 1 910 314 B1**
(45) Date of publication and mention of the grant of the patent: **15.09.2010**
(21) Application number: 06762705.9
(22) Date of filing: 19.07.2006
(51) Int. Cl.: C07D 235/30, C07D 401/12, C07D 403/04, C07D 405/12, A61K 31/4184, A61K 31/4439, A61P 11/08

(54) **BENZIMIDAZOLE DERIVATIVES FOR TREATMENT OF INFLAMMATORY DISEASES**
BENZIMIDAZOLDERIVATE ZUR BEHANDLUNG ENTZÜNDLICHER ERKRANKUNGEN
DERIVES DE BENZIMIDAZOLE DESTINES AU TRAITEMENT DE MALADIES INFLAMMATOIRES

(30) Priority: 21.07.2005 GB 0515025
(43) Date of publication of application: 16.04.2008
(73) Proprietor: Novartis AG, 4056 Basel (CH)
(72) Inventor: BROWN, Lyndon Nigel, Horsham, West Sussex RH12 5AB (GB); MCCARTHY, Clive, CH-4056 Basel (CH); PRESS, Neil John, Horsham, West Sussex RH12 5AB (GB); SOLDERMANN, Nicolas, F-68128 Rosenau (FR)
(74) Representative: Lettenbichler, Isolde Maria
(86) International application number: PCT/EP2006/007110
(87) International publication number: WO 2007/009774

(56) References cited:
- WO-A-02/092568
- WO-A-03/041708
- WO-A-2005/044793
- WO-A-2005/070906

## Description

This invention relates to organic compounds, their preparation and use as pharmaceuticals.

International Patent application No. WO 03/41708 describes kinase inhibitors which can be used to treat COPD based upon 2-acylamino-benzimidazoles. However, the compounds described herein do not have an acyl moiety on the 2-amino group. Furthermore, the compounds described herein have a free carboxy group at the 7-position instead of a carboxamide at the 5- or 6- position of the benzimidazole ring.

International Patent application No. WO 2005/044793 describes a 2-phenylamino-benzimidazole-7-carboxamide prepared from the corresponding 7-carboxylic acid methyl ester. However, the 2-phenylamino-benzimidazole-7-carboxamide is disclosed as having CRF antagonist activity, not CXCR2 receptor antagonist activity. Furthermore, no pharmaceutical activity is disclosed for the intermediate 7-carboxylic acid methyl ester.

International Patent application No. WO 02/92568 describes CXCR2 antagonists, but the antagonists disclosed therein are 2-aryl indoles and are not structurally close to the compounds described herein.

International Patent application No. WO 2005/070906 describes CXCR2 antagonists, but the antagonists disclosed therein are 5-cyano-2-aryl indoles or 5-cyano-2-thiophenyl indoles and are not structurally close to the compounds described herein.

In one aspect the invention provides compounds of formula I 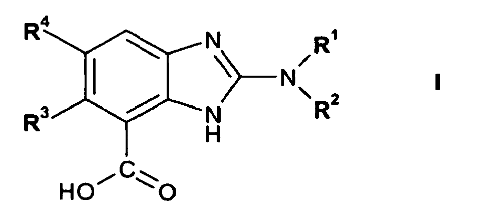 in free or salt form, where
R¹ is hydrogen or C₁-C₈₋alkyl;
R² is phenyl substituted at one position by fluoro and at another position by fluoro, chloro or C₁-C₈-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂ C₁-C₈-alkylthio optionally substituted by C₆-C₁₀-aryl, C₁-C₈-alkylsulfinyl optionally substituted by C₆-C₁₀-aryl, C₁-C₈-alkylsulfonyl optionally substituted by C₆-C₁₀-aryl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is a 5- or 6-membered heterocyclic group containing at least one ring heteroatom selected from nitrogen, oxygen and sulphur, R³ and R⁴ are independently hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio optionally substituted by C₆-C₁₀-aryl, C₁-C₈-alkylsulfinyl optionally substituted by C₆-C₁₀-aryl, C₁-C₈-alkylsulfonyl optionally substituted by C₆-C₁₀-aryl, C₁-C₈-alkoxy, C₁-C₈-haloalkoxy, C₃-C₈-cycloalkyl, C₃-C₈-cycloalkyl-C₁-C₈-alkyl, C₃-C₈-cycloalkyloxy, -CO-NR⁵R⁶, -NH-SO₂R⁷, -NH-COH, -SO₂NH₂ or a 5- or 6-membered heterocyclic group containing at least one ring heteroatom selected from nitrogen, oxygen and sulphur,
or R³ and R⁴ together form a 5- or 6-membered heterocyclic group containing at least one ring heteroatom selected from nitrogen, oxygen and sulphur, and R⁵, R⁶ and R⁷ are independently hydrogen or C₁-C₈-alkyl;
or R² is C₁-C₈-alkyl optionally substituted by a 5- or 6-membered heterocyclic group containing at least one ring heteroatom selected from nitrogen, oxygen and sulphur, R³ and R⁴ are independently hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio optionally substituted by C₆-c₁₀-aryl, C₁-C₈-alkylsulfinyl optionally substituted by C₆-C₁₀-aryl, C₁-C₈-alkylsulfonyl optionally substituted by C₆-C₁₀-aryl, C₁-C₈-alkoxy, C₁-C₈-haloalkoxy, C₃-C₈-cycloalkyl, C₃-C₈-cycloalkyl-C₁-C₈-alkyl, C₃-C₈-cycloalkyl-ox_{y}, -CO-NR⁵R⁶, -NH-SO₂R⁷, -NH-COH, - SO₂NH₂, or a 5- or 6-membered heterocyclic group containing at least one ring heteroatom selected from nitrogen, oxygen and sulphur, or R³ and R⁴ together form a 5- or 6-membered heterocyclic group containing at least one ring heteroatom selected from nitrogen, oxygen and sulphur, and R⁵, R⁶ and R⁷ are independently hydrogen or C₁-C₈-alkyl.

Terms used in the specification have the following meanings:
"Optionally substituted" means the group referred to can be substituted at one or more positions, preferably one or two positions, by any one or any combination of the radicals described.
"C₁-C₈-alkyl" as used herein denotes straight chain or branched alkyl having 1 to 8 carbon atoms. Preferably, C₁-C₈-alkyl is C₁-C₄-alkyl.
"C₂-C₈-alkenyl" as used herein denotes straight chain or branched hydrocarbon chains that contain two to eight carbon atoms and one or more carbon-carbon double bonds. Preferably "C₂-C₈-alkenyl" is "C₂-C₄-alkenyl".
"C₂-C₈-alkynyl"' as used herein denotes straight chain or branched hydrocarbon chains that contain two to eight carbon atoms and one or more carbon-carbon triple bonds. Preferably "C₂-C₈-alkynyl" is "C₂-C₄-alkynyl".
"C₃-C₈-cycloalkyl" as used herein may be, for example, cyclopropyl, cyclobutyl, cyclopentyl, methylcyclopentyl, cyclohexyl, methylcyclohexyl, dimethylcyclohexyl, cycloheptyl, bicycloheptyl, cyclooctyl and bicyclooctyl. Preferably "C₃-C₈-cycloalkyl" is "C₃-C₆-cycloalkyl".
"C₃-C₈-cycloalkyl-C₁-C₈-alkyl" as used herein denotes C₁-C₈-alkyl as hereinbefore defined substituted by C₃-C₈-cycloalkyl as hereinbefore defined. Preferably "C₃-C₈-cycloalkyl-C₁-C₈-alkyl" is "C₃-C₆-cycloalkyl-C₁-C₄-alkyl".
"C₁-C₈-alkylthio" as used herein denotes C₁-C₈-alkyl as hereinbefore defined linked to -S-. Preferably "C₁-C₈-alkylthio" is "C₁-C₄-alkylthio".
"C₁-C₈-alkylsulfinyl" as used herein denotes C₁-C₈-alkyl as hereinbefore defined linked to -S(=O)-. Preferably "C₁-C₈-alkylsulfinyl" is "C₁-C₄-alkysulfinyl".
"C₁-C₈-alkylsulfonyl" as used herein denotes C₁-C₈-alkyl as hereinbefore defined linked to -S(=O)₂-, Preferably "C₁-C₈-alkylsulfonyl" is "C₁-C₄-alkysulfonyl".
"C₆-C₁₀-aryl" as used herein denotes a monovalent carbocyclic aromatic group that contains 6 to 10 carbon atoms and which may be, for example, a monocyclic group such as phenyl or a bicyclic group such as naphthyl. Preferably C₆-C₁₀-aryl is C₆-C₈-aryl, especially phenyl.
"Halo" or "halogen" as used herein denotes a element belonging to group 17 (formerly group VII) of the Periodic Table of Elements, which may be, for example, fluorine, chlorine, bromine or iodine. Preferably halo or halogen is chlorine or fluorine.
"5- or 6- membered heterocyclic group containing at least one ring heteroatom selected from nitrogen, oxygen and sulphur" as used herein may be, for example, pyrrole, pyrrolidine, pyrazole, imidazole, triazole, tetrazole, thiadiazole, isothiazole, oxadiazole, pyridine, oxazole, isoxazole, pyrazine, pyridazine, pyrimidine, piperazine, morpholino, triazine, oxazine, furanyl or thiazole. The heterocyclic group, which is preferably aromatic, is unsubstituted or is substituted by halo, cyano, hydroxy, carboxy, nitro, amido, C₆-C₁₀-aryl, C₁-C₈-alkyl, or C₁-C₈-alkoxy optionally substituted by aminocarbonyl.
"C₃-C₈-cycloalkyl-oxy" as used herein denotes C₃-C₈-cycloalkyl as hereinbefore defined linked to an oxo group. Preferably "C₃-C₈-cycloalkyl-oxy" is "C₃-C₆-cydoalkyl-oxy".
"C₁-C₈-haloalkyl" as used herein denotes C₁-C₈-alkyl as hereinbefore defined substituted by one or more halogen atoms, preferably one, two or three halogen atoms. Preferably "C₁-C₈-haloalkyl" is "C₁-C₄-haloalkyl".
"C₁-C₈-alkoxy" as used herein denotes straight chain or branched alkoxy having 1 to 8 carbon atoms. Preferably, C₁-C₈-alkoxy is C₁-C₄-alkoxy.
"C₁-C₈-haloalkoxy" as used herein denotes C₁-C₈-alkoxy as hereinbefore defined substituted by one or more halogen atoms, preferably one, two or three halogen atoms. Preferably "C₁-C₈-haloalkoxy" is "C₁-C₄-haloalkoxy".
"Carboxy-C₁-C₈-alkoxy" as used herein denotes C₁-C₈-alkoxy as hereinbefore defined substituted by one or more carboxy groups, preferably one or two carboxy groups. Preferably "carboxy-C₁-C₈-alkoxy" is "carboxy-C₁-C₄-alkoxy".
"Aminocarbonyl" as used herein denotes amino attached through the nitrogen atom to a carbonyl group.

Throughout this specification arid in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

Preferred compounds include those of formula I in free or salt form, where
R¹ is hydrogen;
R² is phenyl substituted at one position by fluoro and at another position by fluoro, chloro or C₁-C₈-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂, C₁-C₈-alkylthio optionally substituted by C₆-C₁₀-aryl, C₁-C₈-alkylsulfonyl optionally substituted by C₆-C₁₀-aryl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is a 5- or 6-membered heterocyclic group containing at least one ring heteroatom selected from nitrogen and oxygen, and R³ and R⁴ are both hydrogen;
or R² is C₁-C₈-alkyl optionally substituted by a 5- or 6-membered heterocyclic group containing at least one ring heteroatom selected from nitrogen, oxygen and sulphur, and R³ and R⁴ are independently hydrogen or halo.

Further preferred compounds include those of formula I in free or salt form, where
R¹ is hydrogen;
R² is phenyl substituted at one position by fluoro and at another position by fluoro, chloro or C₁-C₄-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂ C₁-C₄-alkylthio optionally substituted by phenyl, C₁-C₄-alkylsulfonyl optionally substituted by phenyl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is a 5- or 6-membered heterocyclic group containing at least one ring heteroatom selected from nitrogen and oxygen, and R³ and R⁴ are both hydrogen;
or R² is C₁-C₄-alkyl optionally substituted by a 5- or 6-membered heterocyclic group containing at least one oxygen as the heteroatom, and R³ and R⁴ are independently hydrogen or halo.

Especially preferred compounds include those of formula I in free or salt form, where
R¹ is hydrogen;
R² is phenyl substituted at one position by fluoro and at another position by fluoro, chloro or C₁-C₄-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂ C₁-C₄-alkylthio optionally substituted by phenyl, C₁-C₄-alkylsulfonyl optionally substituted by phenyl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is C₁-C₄-alkyl optionally substituted by a 5- or 6-membered heterocyclic group containing at least one oxygen as the heteroatom, and R³ and R⁴ are independently hydrogen or halo.

The compounds represented by formula I can form acid addition salts, particularly pharmaceutically acceptable acid addition salts. Pharmaceutically acceptable acid addition salts of the compound of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula I by known salt-forming procedures.

Compounds of formula I which contain acidic, e.g. carboxyl, groups, are also capable of forming salts with bases, in particular pharmaceutically acceptable bases such as those well known in the art; suitable such salts include metal salts, particularly alkali metal or alkaline earth metal salts such as sodium, potassium, magnesium or calcium salts, or salts with ammonia or pharmaceutically acceptable organic amines or heterocyclic bases such as ethanolamines, benzylamines or pyridine. These salts may be prepared from compounds of formula I by known salt-forming procedures.

Specific especially preferred compounds of the invention are those described hereinafter in the Examples.

The invention also provides a process for the preparation of compounds of formula I which comprises
(i) (A) for the preparation of compounds of formula I, cyclising a compound of formula IIa or IIb in either case optionally in protected form 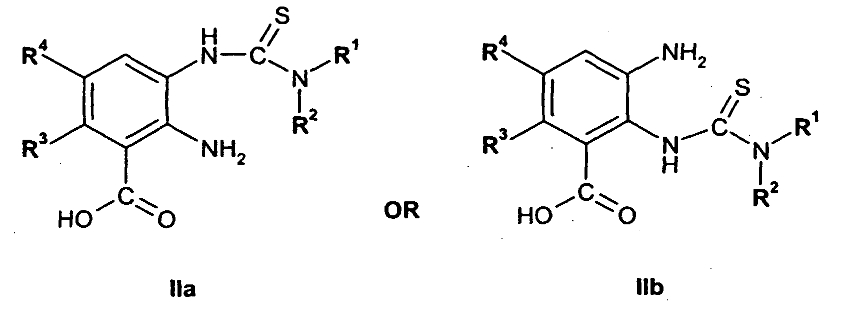 where R¹, R² , R³, and R⁴ are as hereinbefore defined, and deprotecting where necessary;
   (B) for the preparation of compounds of formula I where R¹ is hydrogen, reacting a compound of formula IIa or IIIb in either case optionally in protected form 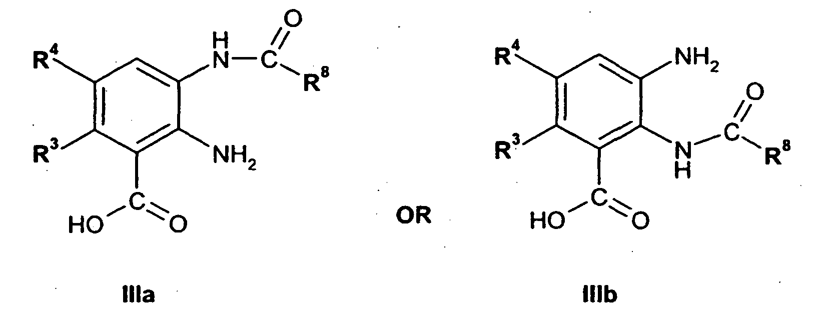 where R³ and R⁴ are as hereinbefore defined and R⁵ is C₁-C₄-alkyl, with a compound of formula IV

   S=C=N-R² IV

   where R² is as hereinbefore defined in the presence of a coupling agent, and deprotecting where necessary; or
   (C) for the preparation of compounds of formula I where at least one of R³ and R⁴ is C₁-C₈-alkylsulfinyl or C₁-C₈-alkylsulfonyl in either case optionally substituted by C₆-C₁₀-aryl, oxidising a compound of formula I where R¹ and R² are as hereinbefore defined, and at least one of R³ and R⁴ as hereinbefore defined is C₁-C₈-alkylthio optionally substituted by C₆-C₁₀-aryl, or a protected form thereof, and deprotecting where necessary; and
(ii) recovering the product in free or salt form.

Process variant (A) may be effected using known procedures for cyclising o-aminophenyl-thioureas to form benzoimidazoles or analogously as hereinafter described in the Examples. The reaction is conveniently carried out in an organic solvent, for example ethanol or acetonitrile in the presence of a coupling agent, for example 1-(-3-dimethylamino-propyl)-3-ethylcarbodiimide hydrochloride (also known as N-(3-dimethylaminopropyl)-N'-ethylcarbodiimide hydrochloride or EDCI). The reaction is carried out at an elevated temperature, for example between 70° C and 120° C, but conveniently at about 80° C or reflux temperature.

Process variant (B) may be effected using known procedures for condensing aromatic diamines with thioisocyanates, or analogously as hereinafter described in the Examples. The coupling agent is preferably 1-(-3-dimethylamino-propyl)-3-ethylcarbodiimide hydrochloride (also known as N-(3-dimethylaminopropyl)-N'-ethylcarbodiimide hydrochloride or EDCI). The reaction is conveniently carried out in an organic solvent, for example acetonitrile. Suitable reaction temperatures are from 10° C to 40° C, for example room temperature.

Process variant (C) may be effected using known procedures for oxidising sulfanyl groups to form sulfinyl or sulfonyl groups or analogously e.g. as hereinafter described in the Examples. The oxidising agent used is preferably a perbenzoic acid, especially meta chloro-per-benzoic acid, e.g. 3-chloro-benzenecarboperoxoic acid. The reaction is conveniently carried out in an organic solvent such as dichloromethane. The reaction temperature may be e.g. from 5 to 25 °C, preferably about 15 °C.

Compounds of formula IIa or IIb may be prepared by reacting a compound of formula Va or Vb respectively 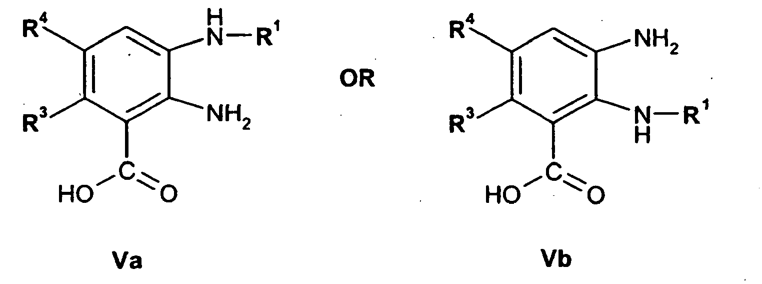 where R¹, R³ and R⁴ are as hereinbefore defined, with a compound of formula IV where R² is as hereinbefore defined using known methods for condensing aromatic diamines with thioisocyanates, or analogously as hereinafter described in the Examples. The reaction is conveniently carried out in an organic solvent, for example ethanol. Suitable reaction temperatures are from 10° C to 40° C, for example room temperature.

Compounds of formula IIIa or IIIb are known or may be prepared by reacting the corresponding nitro-phenylamine with a suitable reducing agent.

Compounds of formula IV are known or may be prepared by known procedures.

Compounds of formula Va or Vb are known or may be prepared by reacting the corresponding nitro-phenylamine with a suitable reducing agent.

Where reference is made herein to protected functional groups or to protecting groups, the protecting groups may be chosen in accordance with the nature of the functional group, for example as described in Protective Groups in Organic Synthesis, T.W. Greene and P.G.M. Wuts, John Wiley & Sons Inc, Third Edition, 1999, which reference also describes procedures suitable for replacement of the protecting groups by hydrogen.

Compounds of formula I in free form may be converted into salt form, and vice versa, in a conventional manner. The compounds in free or salt form can be obtained in the form of hydrates or solvates containing a solvent used for crystallization. Compounds of formula I can be recovered from reaction mixtures and purified in a conventional manner. Isomers, such as enantiomers, may be obtained in a conventional manner, e.g. by fractional crystallization or asymmetric synthesis from correspondingly asymmetrically substituted, e.g. optically active, starting materials.

Compounds of formula I in free or pharmaceutically acceptable salt form, hereinafter referred to alternatively as agents of the invention, are useful as pharmaceuticals. Accordingly the invention also provides a compound of formula I in free or pharmaceutically acceptable salt form for use as a pharmaceutical. The agents of the invention act as CXCR2 receptor antagonists, thereby inhibiting the infiltration and activation of inflammatory cells, in particular neutrophils, monocytes and CD8+ T cells and mediators involved in chronic obstructive pulmonary disease (COPD). The agents of the invention therefore provide symptomatic relief and reduce disease progression.

The airways of subject with COPD exhibit an inflammatory response which is predominantly neutrophilic. When the airways are exposed to cigarette smoke macrophages, CD8+ T cells and epithelial cells are activated and release pro-inflammatory mediators, oxidants, cytokines and neutophilic chemotactic factors, IL-8, GROα, ENA-78 and leukotrienes. IL-8, GROα and ENA-78 are selective chemoattractants for neutrophils. In human neutrophils IL-8 binds two distinct receptors with similar affinity, CXCR1 and CXCR2. Closely related chemokines including GROα, β, y, NAP-2 and ENA-78 bind only to CXCR2. Inhibiting neutrophil recruitment is therefore a recognised therapeutic strategy for treating several lung diseases. Blocking the binding of IL-8, GROα and ENA-78 to the chemokine receptor CXCR2 can provide beneficial effects in patients with COPD by suppressing the infiltration and activation of key inflammatory cells, thereby reducing subsequent tissue damage, mucus secretion, airflow obstruction and disease progression.

The IL-8 and GROα chemokine inhibitory properties of agents of the invention can be demonstrated in the following assays:

### Receptor Binding Assay

[¹²⁵I] IL-8 (human recombinant) was obtained from Amersham Pharmacia Biotech., with specific activity 2000 Ci/mmol. All other chemicals were of analytical grade. Human recombinant CXCR2 receptor expressed in Chinese hamster ovary cells (CHO-K1) was purchased from Euroscreen. The Chinese hamster ovary membranes were prepared according to protocol supplied by Euroscreen. Membrane protein concentration was determined using a Bio-Rad protein assay. Assays were performed in a 96-well micro plate format according the method described in White, et al., J Biol Chem., 1998, 273, 10095). Each reaction mixture contained 0.05 mg/ml CXCR2 membrane protein in 20 mM Bis-Tris-propane, pH 8.0, containing 1.2 mM MgSO_{4,} 0.1 mM EDTA, 25 mM NaCl and 0.03% CHAPS. In addition, compound of interest was added which had been pre-dissolved in dimethylsulphoxide (DMSO) so as to reach a final concentration of between 10 µM and 0.0005 µM (final concentration of DMSO 2 % (v/v)). Binding was initiated by addition of 0.02 nM 125I-IL-8. After 2 hours at room temperature the plate was harvested using a Brandell™ 96-well harvester onto glass fibre filter plate (GF/c) blocked with 1% polyethyleneimine + 0.5% BSA and washed 3 times with 25 mM NaCl, 10 mM TrisHCl, 1 mM MgSO₄, 0.5 mM EDTA, 0.03% CHAPS, pH 7.4. The filter was dried at 50°C overnight. Backseal was then applied to the plate and 50 µl of liquid scintillation fluid added. The counts were then measured on the Packard Topcount™ scintillation counter.

### [³⁵S]-GTPyS binding assay for human CXCR2 receptor using SPA technology

[³⁵S]-GTPyS (with specific activity 1082 Ci/mmol) and wheat germ agglutinin poly vinyl toluene scintillation proximity beads were purchased from Amersham Pharmacia Biotech. The Chinese hamster ovary cell (CHO-K1) membranes expressing human CXCR2 receptors were purchased from Biosignal Packard Inc. All other chemicals were of analytical grade. White non-binding surface 96 well Optiplate™ microplates were obtained from Packard. Recombinant human IL-8 was synthesised, cloned and expressed in *Escherichia coli* as described previously (Lindley I, et al., Proc. Natl. Acad. Sci., 1988, 85(23):9199).

The assay was performed in duplicate in 96 well Optiplate™ microplate in a final volume of 250 µl per well. Compounds were diluted in DMSO (0.5% final concentration) and incubated in 20 mM HEPES buffer pH 7.4 containing 10 mM MgCl₂,100 mM NaCl, 1 mM EDTA plus 100 nM IL-8, 50 µM GDP and 500 pM [³⁵S]GTPyS per well. SPA beads (1 mg/well final concentration) were pre-mixed with the membranes (10 µg/well final concentration) in assay buffer: 20 mM HEPES buffer pH 7.4 containing 10 mM MgCl₂, 100 mM NaCl, 1 mM EDTA. The bead membrane mixture was then added to each well, plates were sealed and incubated at room temperature for 60 minutes. After which time the plate was centrifuged and immediately read on Packard TopCount™ scintillation counter, program [³⁵S dpm] for 1 min/well. Data was expressed as the % response to 100 nM IL-8 minus basal.

### Chemotaxis Assay

The *in vitro* inhibitory properties of these compounds are determined in the neutrophil chemotaxis assay. Assays were performed in a 96-well plate format according to previously published method (Frevert C W, et al., J Immunolog. Methods, 1998, 213, 41). 96-well chemotaxis chambers 5 µm were obtained from Neuro Probe, all cell buffers were obtained from Invitrogen Paisley, UK, dextran -T500 and Ficoll-Paque Plus™ density gradient centrifugation media were purchased from Pharmacia Biotech Buckinghamshire, UK. Calcein-AM dye was obtained from Molecular Probes. Neutrophils were isolated as previously described (Haslett, C., et al. Am J Path., 1985, 119:101). Citrated whole blood was mixed with 4% (w/v) dextran-T500 and allowed to stand on ice for 30 minutes to remove erythrocytes. Granulocytes (PMN) were separated from peripheral blood mononuclear cells by layering 15 ml of cell suspension onto 15 ml Ficoll-Paque PLUS density gradient and centrifuged at 250 xg for 25 minutes. Following centrifugation any erythrocytes contamination of PMN pellet was removed by hypotonic shock lysis using 10 ml ice-cold endotoxin-free sterile water for 50 seconds and neutralised with 10 ml of cold 2x phosphate buffered saline. Isolated neutrophils (1 x10⁷) were then labelled with the fluorochrome calcein-AM (5 µg) in a total volume of 1 ml and incubated for 30 minutes at 37°C. The labelled cells were washed with RPMI without phenol red +0.1% bovine serum albumin, prior to use the cells were counted and adjusted to a final concentration of 5 x 10⁶ cells /ml. The labelled neutrophils were then mixed with test compounds (0.001-1000 nM) diluted in DMSO (0.1% final concentration) and incubated for 10 minutes at room temperature. The chemoattractants (29 µl) are placed in the bottom chamber of a 96-well chemotaxis chamber at a concentration between (0.1-5 nM). The polycarbonate filter (5 µm) was overlaid on the plate, and the cells (25 µl) were loaded on the top filter. The cells were allowed to migrate for 90 minutes at 37° C in a humidified incubator with 5% CO_{2.} At the end of the incubation period, migrated cells were quantified using a multi-well fluorescent plate reader (Fluroskan II™, Labsystems) at 485 nm excitation and 538 nm emission. Each compound is tested in quadruplet using 4 different donors. Positive control cells, i.e. cells that have not been treated with compound, are added to the bottom well. These represent the maximum chemotactic response of the cells. Negative control cells, i.e. those that have not been stimulated by a chemoattractant, are added to the bottom chamber. The difference between the positive control and negative control represents the chemotactic activity of the cells.

Having regard to their inhibition of binding of CXCR2, agents of the invention are useful in the treatment of conditions mediated by CXCR2, for example inflammatory or allergic conditions, particularly chronic obstructive pulmonary airways or lung disease (COPD, COAD or COLD), including chronic bronchitis or dyspnea associated therewith, emphysema, and severe asthma. Treatment in accordance with the invention may be symptomatic or prophylactic.

Prophylactic efficacy in the treatment of chronic bronchitis or COPD will be evidenced by reduced frequency or severity, will provide symptomatic relief and reduce disease progression, improvement in lung function. It may further be evidenced by reduced requirement for other, symptomatic therapy, i.e. therapy for or intended to restrict or abort symptomatic attack when it occurs, for example anti-inflammatory (e.g. corticosteroid) or bronchodilatory.

Other inflammatory or obstructive airways diseases and conditions to which the present invention is applicable include acute lung injury (ALI), acute/adult respiratory distress syndrome (ARDS), idiopathic pulmonary fibrosis, fibroid lung, airway hyperresponsiveness, dyspnea, pulmonary fibrosis, allergic airway inflammation, small airway disease, lung carcinoma, acute chest syndrome in patients with sickle cell disease and pulmonary hypertension, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Agents of the invention are also useful for treating respiratory viral infections, which exacerbate underlying chronic conditions such as asthma, chronic bronchitis, COPD, otitis media, and sinusitis. The respiratory viral infection treated may be associated with secondary bacterial infection, such as otitis media, sinusitis or pneumonia.

Agents of the invention are also useful in the treatment of inflammatory conditions of the skin, for example psoriasis, atopic dermatitis, lupus erythematosus, and other inflammatory or allergic conditions of the skin.

Agents of the invention may also be used for the treatment of other diseases or conditions, in particular diseases or conditions having an inflammatory component, for example, diseases affecting the nose including allergic rhinitis, e.g. atrophic, chronic, or seasonal rhinitis, inflammatory conditions of the gastrointestinal tract, for example inflammatory bowel disease such as ulcerative colitis and Crohn's disease, diseases of the bone and joints including rheumatoid arthritis, psoriatic arthritis, and other diseases such as atherosclerosis, multiple sclerosis, and acute and chronic allograft rejection, e.g. following transplantation of heart, kidney, liver, lung or bone marrow.

Agents of the invention are also useful in the treatment of endotoxic shock, glomerulonephritis, cerebral and cardiac ischemia, Alzheimer's disease, cystic fibrosis, virus infections and the exacerbations associated with them, acquired immune deficiency syndrome (AIDS), multiple sclerosis (MS), *Helicobacter pylori* associated gastritis, and cancers, particularly the growth of ovarian cancer.

Agents of the invention are also useful for treating symptoms caused by viral infection in a human which is caused by the human rhinovirus, other enterovirus, coronavirus, herpes viruses, influenza virus, parainfluenza virus, respiratory syncytial virus or an adenovirus.

The effectiveness of an agent of the invention in inhibiting inflammatory conditions, for example in inflammatory airways diseases, may be demonstrated in an animal model, e.g. mouse, rat or rabbit model, of airway inflammation or other inflammatory conditions, for example as described by Wada et al, J. Exp. Med (1994) 180:1135-40; Sekido et al, Nature (1993) 365:654-57; Modelska et al., Am. J. Respir. Crit. Care. Med (1999) 160:1450-56; and Laffon et al (1999) Am. J. Respir. Crit. Care Med. 160:1443-49.

The agents of the invention are also useful as co-therapeutic agents for use in combination with other drug substances such as anti-inflammatory, bronchodilatory, antihistamine or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance. Accordingly the invention includes a combination of an agent of the invention as hereinbefore described with an anti-inflammatory, bronchodilatory, antihistamine or anti-tussive drug substance, said agent of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTB4 antagonists such as BIIL 284, CP-195543, DPC11870, LTB4 ethanolamide, LY 293111, LY 255283, CGS025019C, CP-195543, ONO-4057, SB 209247, SC-53228 and those described in US 5451700; LTD4 antagonists such include montelukast, pranlukast, zafirlukast, accolate, SR2640, Wy-48,252, ICI 198615, MK-571, LY-171883, Ro 24-5913 and L-648051; PDE4 inhibitors such cilomilast (Ariflo® GlaxoSmithKiine), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; A_{2A} agonists such as those described in EP 1052264, EP 1241176, EP 409595A2, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, and WO 03/086408; and A_{2B} antagonists such as those described in WO 02/42298.

Suitable bronchodilatory drugs include anticholinergic or antimuscarinic agents, in particular ipratropium bromide, oxitropium bromide, tiotropium salts and CHF 4226 (Chiesi), and glycopyrrolate, but also those described in EP 424021, US 3714357, US 5171744, WO 01/04118, WO 02/00652, WO 02/51841, WO 02/53564, WO 03/00840, WO 03/33495, WO 03/53966, WO.03/87094, WO 04/018422 and WO 04/05285; and beta-2 adrenoceptor agonists such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, which document is incorporated herein by reference, preferably compounds of the Examples thereof, especially a compound of formula 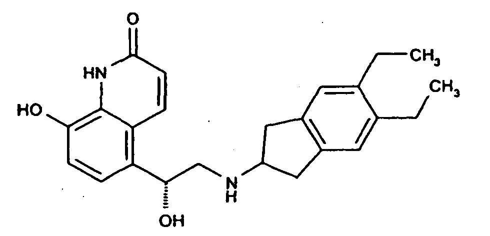 and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 , WO 04/80964, EP1460064, WO 04/087142, WO 04/089892, EP 01477167, US 2004/0242622, US 2004/0229904, WO 04/108675, WO 04/108676, WO 05/033121, WO 05/040103 and WO 05/044787.

Suitable dual anti-inflammatory and bronchodilatory drugs include dual beta-2 adrenoceptor agonist / muscarinic antagonists such as those disclosed in US 2004/0167167, WO 04/74246 and WO 04/74812.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in JP 2004107299, WO 03/099807 and WO 04/026841.

Combinations of agents of the invention and anticholinergic or antimuscarinic agents, steroids, beta-2 agonists, PDE4 inhibitors, dopamine receptor agonists, LTD4 antagonists or LTB4 antagonists may also be used. Other useful combinations of agents of the invention with anti-inflammatory drugs are those with other antagonists of chemokine receptors, e.g. CCR-1, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzocydohepten-8-yl]carbonyl]amino]phenyl]-methyl]-tetrahydro-N,N-dimethyl-2H-pyran-4-aminium chloride (TAK-770), CCR-5 antagonists described in US 6166037 (particularly claims 18 and 19), WO 0066558 (particularly claim 8), WO 0066559 (particularly claim 9), WO 04/018425 and WO 04/026873.

In accordance with the foregoing, the invention also provides a method for the treatment of a condition mediated by CXCR2, for example an inflammatory or allergic condition, particularly an inflammatory or obstructive airways disease, which comprises administering to a subject, particularly a human subject, in need thereof an effective amount of a compound of formula I in a free or pharmaceutically acceptable salt form as hereinbefore described. In another aspect the invention provides the use of a compound of formula I, in free or pharmaceutically acceptable salt form, as hereinbefore described for the manufacture of a medicament for the treatment of a condition mediated by CXCR2, for example an inflammatory or allergic condition, particularly an inflammatory or obstructive airways disease.

The agents of the invention may be administered by any appropriate route, e.g. orally, for example in the form of a tablet or capsule; parenterally, for example intravenously; by inhalation, for example in the treatment of inflammatory or obstructive airways disease; intranasally, for example in the treatment of allergic rhinitis; topically to the skin, for example in the treatment of atopic dermatitis; or rectally, for example in the treatment of inflammatory bowel disease.

In a further aspect, the invention also provides a pharmaceutical composition comprising as active ingredient a compound of formula I in free or pharmaceutically acceptable salt form, optionally together with a pharmaceutically acceptable diluent or carrier therefor. The composition may contain a co-therapeutic agent such as an anti-inflammatory bronchodilatory or antihistamine drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula I having a particle diameter up to 10 microns, optionally together with a diluent or carrier, such as lactose, of the desired particle size distribution and a compound that helps to protect against product performance deterioration due to moisture, such as magnesium stearate e.g. 0.05 to 1.5%. When the composition comprises a nebulised formulation, it preferably contains, for example, the compound of formula I either dissolved, or suspended, in a vehicle containing water, a cosolvent such as ethanol or propylene glycol and a stabiliser, which may be a surfactant.

The invention includes (A) an agent of the invention in inhalable form, e.g. in an aerosol or other atomisable composition or in inhalable particulate, e.g. micronised form, (B) an inhalable medicament comprising an agent of the invention in inhalable form; (C) a pharmaceutical product comprising such an agent of the invention in inhalable form in association with an inhalation device; and (D) an inhalation device containing an agent of the invention in inhalable form.

Dosages of agents of the invention employed in practising the present invention will of course vary depending, for example, on the particular condition to be treated, the effect desired and the mode of administration. In general, suitable daily dosages for administration by inhalation are of the order of 0.01 to 1 mg/kg per day while for oral administration suitable daily doses are of the order of 0.005 to 100 mg/kg of total body weight. The daily parenteral dosage regimen will preferably be about 0.001 to about 80 mg/kg of total body weight. The daily topical dosage regimen will preferably be from 0.1 mg to 150 mg, administered one to four, preferably two or three times daily.

The invention is illustrated by the following Examples.

The following abbreviations are used:
- RT: room temperature
- aq.: aqueous
- DMSO: dimethylsulfoxide
- THF: tetrahydrofuran

### EXAMPLES

Especially preferred compounds of formula I are shown in the Table I below, the methods of preparation being described hereinafter. All compounds are trifluoroacetic acid salts. The table also shows characterising mass spectrometry data.

**TABLE I**

| Ex. | Structure | MH+ | Ki or Kb (µM) |
|---|---|---|---|
| **1** | 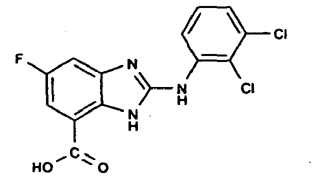 | 340 | 0.2 |
| **2** | 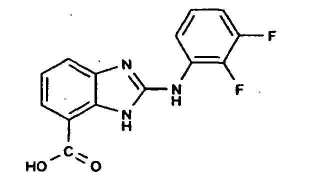 | 289 | - |
| **3** | 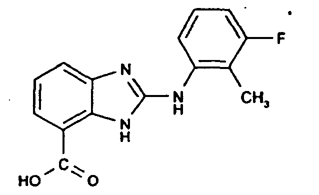 | 285 | 0.8 |
| **4** | 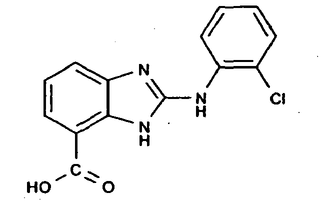 | 288 | - |
| **5** | 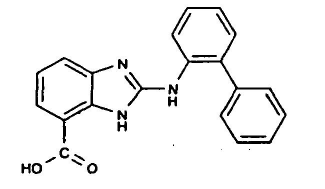 | 329 | 1.2 |
| **6** | 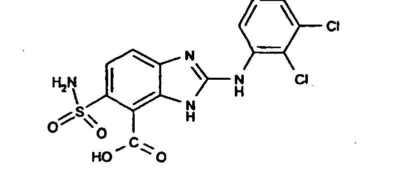 | - | - |
| **7** | 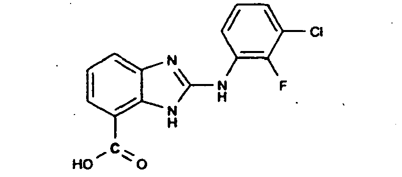 | - | - |
| **8** | 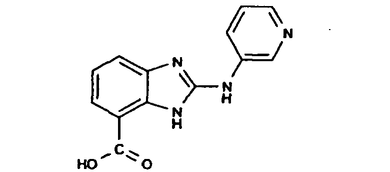 | - | - |
| **9** | 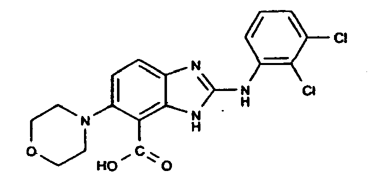 | - | - |
| **10** | 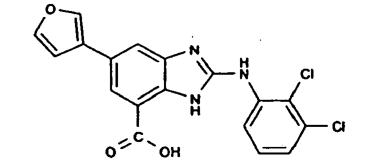 | 388 | 1.5 |
| **11** | 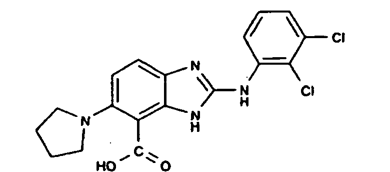 | - | - |
| **12** | 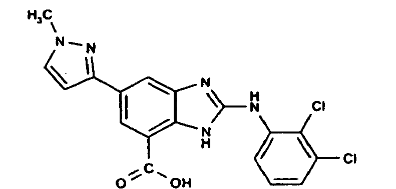 | 402 | 1.8 |
| **13** | 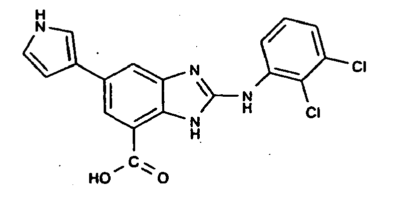 | 387 | - |
| **14** | 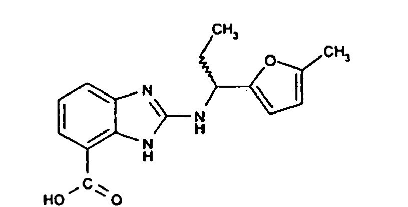 | - | - |
| **15** | 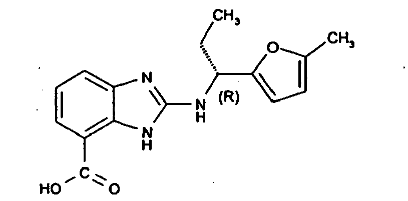 | - | - |
| **16** | 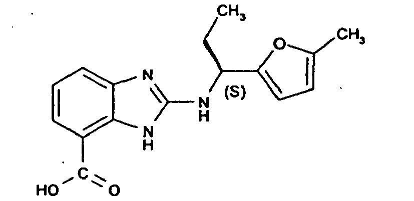 | - | - |
| **17** | 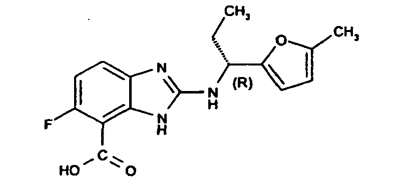 | - | - |
| **18** | 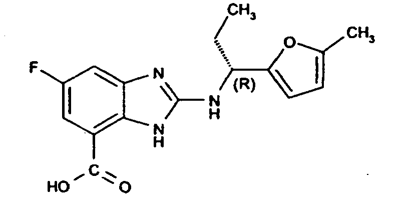 | - | - |
| **19** | 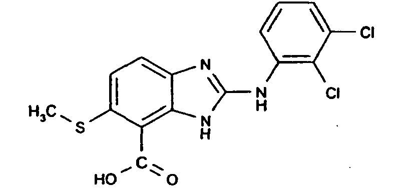 | - | - |
| **20** | 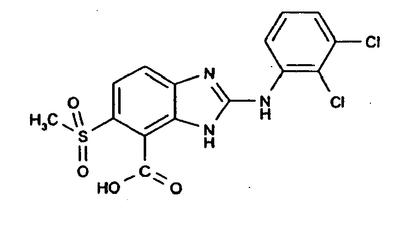 | - | - |
| **21** | 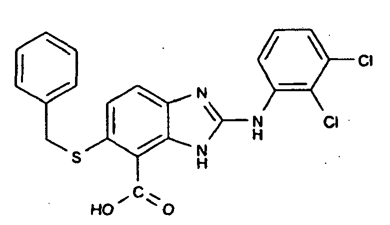 | - | - |
| **22** | 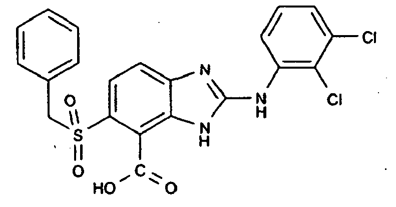 | - | - |

Ki data and Kb data are determined in the aforementioned Receptor Binding and [³⁵S]-GTPyS Binding Assays respectively.

LC specificity: The retention times (Rₜ) are obtained on a Waters HPLC alliance-HT system with a Xterra MS column C18 50/4.6 5µm applying a gradient H₂O (+0.1% formic acid) / CH₃CN (+0.1 % formic acid) 95/5 to 0/100 over 4 minutes and 1.8 mL/minute as solvent flow and 40°C for the oven temperature.

### Example 1

### 2-(2,3-Dichloro-phenylamino)-6-fluoro-3H-benzoimidazole-4-carboxylic acid

### (a) 2-Amino-5-fluoro-benzoic acid methyl ester:

To a solution of 2-amino-5-fluoro-benzoic acid (1 g) in 30 mL of THF/MeOH (2:1) is added dropwise at RT a 2M solution of trimethylsilyldiazomethane (3.4 mL). The reaction mixture obtained is stirred for 3 hours at RT. The reaction is quenched by the addition of few drops of AcOH and diluted with EtOAc. The organic phase obtained is washed with (6N) NaOH aq., brine and H₂O, dried over MgSO₄, filtrated and solvent is evaporated under vacuum to afford the desired product.

### (b) 2-Acetylamino-5-fluoro-3-nitro-benzoic acid methyl ester:

2-Amino-5-fluoro-benzoic acid methyl ester (866 mg) is dissolved in acetic anhydride (4.8 mL) and stirred for 4 hours. A mixture of acetic anhydride, acetic acid and HNO₃ 60% is added dropwise to the solution at RT and the reaction mixture obtained is stirred at RT for 16 hours. The reaction is quenched and neutralized to pH 6-7 with aq. NaOH 6N and extracted with EtOAc. The organic layer obtained is washed with brine, H₂O, dried over MgSO₄ and solvent is evaporated. The resulting oil adsorbed on isolute® sorbent is purified by flash chromatography on silica gel eluting with hexane: ethyl acetate 2:1 to afford the title compound (LC/MS, >90% purity (254 nm), Rₜ=2.57).

### (c) 2-Acerylamino-3-amino-5-fluoro-benzoic acid methyl ester:

To a solution of 2-Acetylamino-5-fluoro-3-nitro-benzoic acid methyl ester (145 mg) in MeOH (15 mL) is added raney-Ni and the reaction mixture obtained is stirred under a H₂ atmosphere. After 5 hours, the reaction is stopped, the mixture obtained is filtered and solvent is evaporated under vacuum to afford the title compound.

### (d) 2-(2,3-Dichloro-phenylamino)-6-fluoro-3H-benzoimidazole-4-carboxylic acid methyl ester:

To a solution of 2-acetylamino-3-amino-5-fluoro-benzoic acid methyl ester (63 mg) in acetonitrile (1.5 mL) are added 2,3-dichlorophenyl isothiocyanate (48 µL) and N-(3-dimethylaminopropyl)-N'-ethylcarbodiimide hydrochloride (EDCI, 81 mg). The reaction mixture obtained is stirred at RT for 16 hours. Solvent is evaporated, the residue obtained is dissolved in CH₂Cl₂, the organic layer obtained is washed with brine, H₂O, dried over MgSO₄, filtrated and solvent is evaporated. 50 mg of this crude solid are purified by RP-C18 chromatography eluting with H₂O (+0.1 % trifluoroacetic acid): acetonitrile (+0.1 % trifluoroacetic acid) gradient (75:25 to 30:70 over 15 minutes) to afford the title compound as a trifluoroacetic acid salt (LC/MS, 100% purity (254nm), Rₜ=6.39, [M]⁺=353.94 and 355.95 and 357.95).

### (e) 2-(2,3-Dichloro-phenylamino)-6-fluoro-3H-benzoimidazole-4-carboxylic acid:

To a stirred solution of 2-(2,3-dichlorophenylamino)-6-fluoro-3H-benzoimidazole-4-carboxylic acid methyl ester (50 mg) in THF (3 mL) is added a solution of LiOH.H₂O (118.5 mg) in H₂O (1.5ml). The reaction mixture obtained is heated to 60°C for 4 hours. The mixture obtained is cooled to RT, quenched with an aq. 1N HCl solution and neutralized to pH 4-5. The aq. phase is extracted with EtOAc and the organic layer obtained is combined and washed with brine, H₂O, dried over MgSO₄, filtrated and solvent is evaporated. The crude solid obtained is purified by RP-C18 chromatography eluting with H₂O (+0.1% trifluoroacetic acid): acetonitrile (+0.1% trifluoroacetic acid) gradient (95: 5 to 50:50 over 10 minutes) to afford the title compound as a trifluoroacetic acid salt (LC/MS, >95% purity, Rₜ=3.04, [M]⁺=339.90 and 341.89 and 343.89).

### Examples 2 to 8

The compounds of these Examples are prepared analogously to Example 1 using appropriate starting materials.

### Example 9

### 2-(2,3-Dichloro-phenylamino)-5-morpholin-4-yl-3H-benzoimidazole-4-carboxylic acid

### (a) 2-Amino-6-morpholin-4-yl-3-nitro-benzoic acid ethyl ester:

To a stirred solution of 2-amino-6-fluoro-3-nitrobenzoic acid ethyl ester (200 mg) in DMSO (2 L) is added morpholin (0.230 L) and the reaction mixture obtained is heated to 100°C overnight. The mixture obtained is quenched with H₂O and extracted with EtOAc. The organic layer obtained is dried over MgSO₄, filtrated and solvent is evaporated to afford the title compound (LC/MS Rₜ=2.79, [M]⁺=296.10 and 297.11).

### (b) 2,3-Diamino-6-morpholin-benzoic acid ethyl ester:

To a stirred solution of 2-amino-6-morpholin-3-nitro-benzoic acid ethyl ester (245 mg) in EtOH (2 mL) is added SnCl₂ anhydrous (722 mg) and the reaction mixture obtained is heated to 70°C for 16 hours. After cooling to RT, solvent is evaporated and the residue obtained is dissolved in H₂O and treated with 40% NaOH aq. to pH 12 and extracted with CH₂Cl₂. The organic layers obtained are combined, dried over MgSO₄, filtrated and solvent is evaporated to afford the title compound (LC/MS, 100% purity, Rₜ=1.42, [M]⁺=266.16 and 267.16).

### (c) 2-(2,3-Dichloro-phenylamino)-5-morpholin-4-yl-3H-benzoimidazole-4-carboxylic acid:

To a solution of 2,3-diamino-6-morpholin-benzoic acid ethyl ester (136 mg) in acetonitrile (2 mL) are added 2,3-dichlorophenyl isothiocyanate (90 µL). The reaction mixture obtained is stirred for 1 hour at RT and N-(3-dimethylaminopropyl)-N'-ethylcarbodiimide hydrochloride (EDCI, 150 mg) is added. The mixture obtained is stirred at RT for 16 hours. The reaction mixture obtained is diluted in THF (2 mL) and a solution of LiOH.H₂O (643 mg) in H₂O (1 ml) is added. The reaction mixture obtained is heated to 60°C for 16 hours: The mixture obtained is cooled to RT, quenched with aq. 1N HCl solution an neutralized to pH 4-5. The aq. phase obtained is extracted with EtOAc and the organic layers obtained are combined and washed with brine, H₂O, dried over MgSO₄, filtrated and solvent is evaporated. Purification by RP-C18 chromatography eluting with H₂O (+0.1% trifluoroacetic acid): acetonitrile (+0.1% trifluoroacetic acid) gradient (95: 5 to 50:50 over 10 minutes) to afford the title compound as a trifluoroacetic acid salt (LC/MS, 100% purity, Rₜ=2.58, [M]⁺=406.95 and 408.94 and 409.93).

### Example 10

This compound is prepared analogously to Example 9 using appropriate starting materials.

### Example 11

### 2-(2,3-Dichloro-phenylamino)-5-pyrrolidin-1-yl-3H-benzoimdazole-4-carboxylic acid

### (a) 2-Amino-3-nitro-6-pyrrolidin-1-yl-benzoic acid ethyl ester:

To a stirred solution of 2-amino-6-fluoro-3-nitrobenzoic acid ethyl ester (200 mg) in DMSO (2 mL) is added pyrrolidine (0.109 mL) and the reaction mixture obtained is heated to 100°C overnight. The mixture obtained is quenched with H₂O and extracted with EtOAc. The organic layer obtained is dried over MgSO₄, filtrated and solvent is evaporated to afford the title compound (LC/MS, 100% purity, Rₜ=3.14, [M+1]⁺=280.12).

### (b) 2,3-Diamino-6- pyrrolidin-benzoic acid ethyl ester:

To a stirred solution of 2-amino-6-pyrolidin-3-nitro-benzoic acid ethyl ester (80 mg) in EtOH (2 mL) is added SnCl₂ anhydrous (554 mg) and the reaction mixture obtained is heated to 70°C for 16 hours. After cooling to RT, solvent is evaporated and the residue obtained is dissolved in H₂O treated with 40% NaOH aq. to pH 12 and extracted with CH₂Cl₂. The organic layers obtained are combined, dried over MgSO_{4,} filtrated and solvent is evaporated to afford the title compound (LC/MS Rₜ=1.50, [M+1]⁺=250.18).

### (c) 2-(2,3-Dichloro-phenylamino)-5-pyrrolidin-1-yl-3H-benzoimidazole-4-carboxylic acid:

To a solution of 2,3-diamino-6-pyrrolidin-benzoic acid ethyl ester (60 mg) in acetonitrile (2 mL) are added 2,3-dichlorophenyl isothiocyanate (32 µL). The reaction mixture obtained is stirred for 1 hour at RT and N-(3-dimethylaminopropyl)-N'-ethylcarbodiimide hydro-chloride (EDCI, 53 mg) is added. The mixture obtained is stirred at RT for 16 hours. The reaction mixture obtained is diluted in THF (2 mL) and a solution of LiOH.H₂O (229 mg) in H₂O (1 ml) is added . The reaction mixture obtained is heated to 60°C for 16 hours. The mixture obtained is cooled to RT, quenched with an aq. 1N HCl solution and neutralized to pH 4-5. The aqueous phase obtained is extracted with EtOAc, the organic layers obtained are combined and washed with brine, H₂O, dried over MgSO₄, filtrated and solvent is evaporated. Purification by RP-C18 chromatography eluting with water (+0.1% trifluoroacetic acid): acetonitrile (+0.1% trifluoroacetic acid) gradient (95: 5 to 50:50 over 10 minutes) to afford the title compound as a trifluoroacetic acid salt (LC/MS, Rₜ=2.41, [M]⁺=390.95 and 392.97 and 393.95).

### Examples 12 to 18

The compounds of these Examples are prepared analogously to Example 11 using appropriate starting materials.

### Example 19

### 2-(2,3-Dichloro-phenylamino)-5-methylsulfanyl-3H-benzoimidazole-4-carboxylic acid

### (a) 2-Amino-6-methylsulfanyl-3-nitro-benzoic acid ethyl ester:

To a stirred solution of 2-amino-6-fluoro-3-nitrobenzoic acid ethyl ester (300 mg) in THF (1 mL) is added sodium methanthiolate (190 mg) and the reaction mixture obtained is heated to 100°C overnight. The mixture obtained is quenched with H₂O and extracted with EtOAc. The organic layer obtained is dried over MgSO₄, filtrated and solvent is evaporated to afford the title compound (LC/MS, Rₜ=3.22, [M-1]=255.1).

### (b) 2,3-Diamino-6-methylsulfanyl-benzoic acid ethyl ester:

To a stirred solution of 2-amino-6-methylsulfanyl-3-nitro-benzoic acid ethyl ester (164 mg) in EtOH (15 mL) is added SnCl₂ anhydrous (557 mg) and the reaction mixture obtained is heated to 70°C for 16 hours. After cooling to Rt, solvent is evaporated and the residue obtained is dissolved in H₂O treated with 40% aq. NaOH to pH 12 and extracted with CH₂Cl₂. The organic layers obtained are combined, dried over MgSO₄, filtrated and solvent is evaporated to afford the title compound (LC/MS Rₜ=2.19, [M+1=227.1).

### (c) 2-(2,3-Dichloro-phenylamino)-5-methylsulfanyl-3H-benzoimidazole-4-carboxylic acid ethyl ester:

To a solution of 2,3-diamino-6-methylsulfanyl-benzoic acid ethyl ester (150 mg) in acetonitrile (10 mL) are added 2,3-dichlorophenyl isothiocyanate (98 µL). The reaction mixture obtained is stirred 1 hour at RT and N-(3-dimethylaminopropyl)-N'-ethylcarbodiimide hydro-chloride (EDGI, 166 mg) is added. The mixture obtained is cooled to RT, quenched with H₂O and extracted with EtOAc. The organic layers obtained are combined, dried over MgSO₄, filtrated and solvent is evaporated to afford the title compound (LC/MS Rₜ=3.55, [M]⁺=395.93 and 397.89 and 399.89).

### (d) 2-(2,3-Dichloro-phenylamino)-5-methylsulfanyl-3H-benzoimidazole-4-carboxylic acid:

To a solution of 2-(2,3-dichloro-phenylamino)-5-methylsulfanyl-3H-benzoimidazole-4-carboxylic acid ethyl ester (50 mg) in THF (2mL) and a solution of LiOH.H₂O (241 mg) in H₂O (1 ml) is added . The reaction mixture obtained is heated to 60°C for 16 hours. The mixture obtained is cooled to RT, quenched with a aq. 1N HCl solution an neutralized to pH 4-5. The aqueous phase obtained is extracted with EtOAc and the organic layers obtained are combined and washed with brine, H₂O, dried over MgSO₄, filtrated and solvent is evaporated.

Purification by RP-C18 chromatography eluting with H₂O (+0.1% trifluoroacetic acid): acetonitrile (+0.1 % trifluoroacetic acid) gradient (95: 5 to 50:50 over 10 minutes) affords the title compound as a trifluoroacetic acid salt (LC/MS, 100% purity, Rₜ=2.81, [M]⁺=367.86 and 369.88 and 371.88).

### Example 20

### 2-(2,3-Dichloro-phenylamino)-5-methanesulfonyl-3H-benzoimidazole-4-carboxylic acid

### (a) 2-(2,3-Dichloro-phenylamino)-5-methanesulfonyl-3H-benzoimidazole-4-carboxylic acid ethyl ester:

To a solution of 2-(2,3-dichloro-phenylamino)-5-methylsulfanyl-3H-benzoimidazole-4-carboxylic acid ethyl ester (57 mg) in CH₂Cl₂ (1 mL) is added a solution of 3-Chloro-benzenecarboperoxoic acid (66 mg) in CH₂Cl₂ (1 mL) at RT and the solution obtained is stirred for 16 hours. The reaction is quenched with NaOH (3eq.) and filtered and solvent is evaporated. Purification by flash chromatography (SiO2, CH₂Cl₂/MeOH, 95/5) afford the title compound (LC/MS, Rₜ=3.22, [M]⁺=427.87 and 431.89).

### (b) 2 -(2,3-Dichloro-phenylamino)-5-methanesulfonyl-3H-benzoimidazole-4-carboxylic acid:

To a solution of 2-(2,3-dichloro-phenylamino)-5-methanesulfonyl-3H-benzoimidazole-4-carboxylic acid ethyl ester (40 mg) in THF (2 mL) is added at RT a solution of LiOH.H₂O (60 mg) in H₂O (1 ml). The reaction mixture obtained is stirred at 60° C for 16 hours. The reaction is quenched with a HCl 1N aq. solution, acidified to pH 5 and extracted with EtOAc. The organic layer obtained is dried over MgSO₄, filtrated and solvent is evaporated. The crude product is purified by RP-C18 chromatography eluting with H₂O (+0.1% trifluoroacetic acid): acetonitrile (+0.1% trifluoroacetic acid) gradient (95: 5 to 50:50 over 10 minutes) to afford the title compound as a trifluoroacetic acid salt (LC/MS, 100% purity, Rₜ=2.55, [M]⁺=399.91 and 403.95).

### Example 21

### 5-Benzylsulfanyl-2-(2,3-dichloro-phenylamino)-3H-benzoimidazole-4-carboxylic acid

### (a) 2-Amino-6-benzylsulfanyl-3-nitro-benzoic acid ethyl ester:

To a stirred solution of 2-amino-6-fluoro-3-nitrobenzoic acid ethyl ester (300 mg) in DMSO (2 mL) is added benzylmercaptan and the reaction mixture obtained is heated to 100°C overnight. The mixture is quenched with H₂O and extracted with EtOAc. The organic layer obtained is dried over MgSO₄, filtrated and solvent is evaporated to afford the title compound (LC/MS, Rₜ=3.70, [M+1]⁺=333.03).

### (b) 2,3-Diamino-6-benzylsulfanyl-benzoic acid ethyl ester:

To a stirred solution of 2-amino-6-benzylsulfanyl-3-nitro-benzoic acid ethyl ester (700 mg) in EtOH (15 mL) is added SnCl₂ anhydrous (1:43 g) and the reaction mixture obtained is heated to 70°C for 16 hours. After cooling to RT, solvent is evaporated and the residue obtained is dissolved in H₂O treated with 40% NaOH aq. to pH 12 and extracted with CH₂Cl₂. The organic layers obtained are combined, dried over MgSO₄, filtrated and solvent is evaporated to afford the title compound (LC/MS, Rₜ=3.04, [M+1]⁺=303.11).

### (c) 5-Benzylsulfanyl-2-(2,3-dichloro-phenylamino)-3H-benzoimidazole-4-carboxylic acid:

To a solution of 2,3-diamino-6-benzylsulfanyl-benzoic acid ethyl ester (660 mg) in acetonitrile (5 mL) are added 2,3-dichlorophenyl isothiocyanate (308 µL). The reaction mixture obtained is stirred 1 hour at RT and N-(3-dimethylaminopropyl)-N'-ethylcarbodiimide hydro-chloride (EDCI, 521 mg) is added. The mixture obtained is stirred at RT for 16 hours. The reaction mixture obtained is diluted in THF (20 mL) and a solution of LiOH.H₂O (2.25 g) in H₂O (10 ml) is added . The reaction mixture obtained is heated to 60°C for 16 hours. The mixture obtained is cooled to RT, quenched with an aq. 1N HCl solution an neutralized to pH 4-5. The aqueous phase obtained is extracted with EtOAc and the organic layers obtained are combined, and washed with brine, H₂O, dried over MgSO₄, filtrated and solvent is evaporated to afford the title compound. A sample of this crude product is taken and is purified by RP-C18 chromatography eluting with H₂O (+0.1% trifluoroacetic acid): acetonitrile (+0.1% trifluoroacetic acid) gradient (95: 5 to 50:50 over 10 minutes) to afford the title compound as a trifluoroacetic acid salt (LC/MS, 100% purity, Rₜ=3.44, [M]⁺=443.83 and 445.86 and 446.87).

### Example 22

### 2-(2,3-Dichloro-phenylamino)-5-phenylmethanesulfonyl-3H-benzoimidazole-4-carboxylic acid

### (a) 2-(2,3-Dichloro-phenylamino)-5-phenylmethanesulfanyl-3H-benzoimidazole-4-carboxylic acid methyl ester:

To a solution of 5-benzylsulfanyl-2-(2,3-dichloro-phenylamino)-3H-benzoimidazole-4-carboxylic acid (80 mg) in THF (2 mL) and MeOH (1mL) is added dropwise a 2N solution in THF of trimethylsilyldiazomethan (0.170 mL) at RT. The reaction mixture obtained is stirred for 2 hours at RT. The reaction is quenched with few drops of AcOH, diluted with EtOAc, washed with 2N NaOH aq., dried over MgSO₄, filtrated and solvent is evaporated. The crude product is purified by flash chromatography (SiO2, hexane/EtOAc, 5/1) to afford the title compound (LC/MS, 100% purity, Rₜ=3.95, [M]⁺=457.84 and 459.84 and 460.85).

### (b) 2-(2,3-Dichloro-phenylamino)-5-phenylmethanesulfonyl-3H-benzoimidazole-4-carboxylic acid methyl ester:

To a solution of 2-(2,3-dichloro-phenylamino)-5-phenylmethanesulfonyl-3H-benzoimidazole-4-carboxylic acid methyl ester (80 mg) in CH₂Cl₂(1 mL) is added a solution of 3-chloro-benzenecarboperoxoic acid (88 mg) in CH₂Cl₂ (1 mL) at RT and the solution obtained is stirred for 16 hours. The reaction is quenched with NaOH (3eq.) and filtered and solvent is evaporated to afford the title compound (LC/MS, Rₜ=3.58, [M]⁺=489.88 and 491.90 and 492.90).

### (c) 2-(2,3-Dichloro-phenylamino)-5-phenylmethanesulfonyl-3H-benzoimidazole-4-carboxylic acid:

To a solution of 2-(2,3-dichloro-phenylamino)-5-phenylmethanesulfonyl-3H-benzoimidazole-4-carboxylic acid methyl ester (56 mg) in THF (2 mL) is added at RT a solution of LiOH.H₂O (131 mg) in H₂O (1 ml). The reaction mixture obtained is stirred at 60° C for 16 hours. The reaction is quenched with a HCl 1N aq. solution, acidified to pH=5 and extracted with EtOAc. The organic layer obtained is dried over MgSO_{4,} filtrated and solvent is evaporated. The crude product is purified by RP-C18 chromatography eluting with H₂O (+0.1% trifluoroacetic acid): acetonitrile (+0.1% trifluoroacetic acid) gradient (95: 5 to 50:50 over 10 minutes) to afford the title compound as a trifluoroacetic acid salt (LC/MS, 100% purity, Rₜ=3.26, [M]⁺=475.84 and 477.79).

## Claims

1. A compound of formula I 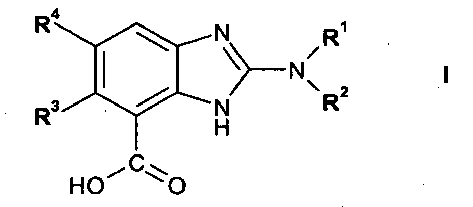 in free or salt form, where
R¹ is hydrogen or C₁-C₈-alkyl;
R² is phenyl substituted at one position by fluoro and at another position by fluoro, chloro or C₁-C₈-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂, C₁-C₈-alkylthio optionally substituted by C₆-C₁₀-aryl, C₁-C₈-alkylsulfinyl optionally substituted by C₆-C₁₀-aryl, C₁-C₈-alkylsulfonyl optionally substituted by C₆-C₁₀-aryl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is a 5- or 6-membered heterocyclic group containing at least one ring heteroatom selected from nitrogen, oxygen and sulphur, R³ and R⁴ are independently hydrogen, halo, cyano, hydroxy, nitro, carboxy, aminocarbonyl, C₁-C₈-alkyl, C₁-C₈-alkenyl, C₁-C₈-alkynyl, C₁₋₈-haloalkyl, C₁-C₈-alkylthio optionally substituted by C₆-C₁₀-aryl, C₁-C₈-alkylsulfinyl optionally substituted by C₆-C₁₀-aryl, C₁-C₈-alkylsulfonyl optionally substituted by C₆-C₁₀-aryl, C₁-C₈-alkoxy, C₁-C₈-haloalkoxy, C₃-C₈-cycloalkyl, C₃-C₈-cycloalkyl-C₁-C₈-alkyl, C₃-C₈-cycloalkyloxy, -CO-NR⁵R⁶, -NH-SO₂R⁷, -NH-COH, -SO₂NH₂, or a 5- or 6-membered heterocyclic group containing at least one ring heteroatom selected from nitrogen, oxygen and sulphur,
or R³ and R⁴ together form a 5- or 6-membered heterocyclic group containing at least one ring heteroatom selected from nitrogen, oxygen and sulphur, and R⁵, R⁶ and R⁷ are independently hydrogen or C₁-C₈-alkyl; or
or R² is C₁-C₈-alkyl optionally substituted by a 5- or 6-membered heterocyclic group containing at least one ring heteroatom selected from nitrogen, oxygen and sulphur, R³ and R⁴ are independently hydrogen, halo, cyano, hydroxy, nitro, carboxy, aminocarbonyl, C₁-C₈-alkyl, C₁-C₈-alkenyl, C₁-C₈-alkynyl, C₁-C₈-haloalkyl, C₁-C₈-alkylthio optionally substituted by C₆-C₁₀-aryl, C₁-C₈-alkylsulfinyt optionally substituted by C₆-C₁₀-aryl, C₁-C₈-alkylsulfonyl optionally substituted by C₆-C₁₀-aryl, C₁-C₈-alkoxy, C₁-C₈-haloalkoxy, C₃-C₈-cycloalkyl, C₃-C₈-cycloalkyl-C₁-C₈-alkyl, C₃-C₈-cycloalkyl-oxy, -CO-NR⁵R⁶, -NH-SO₂R⁷, -NH-COH, - SO₂NH₂, or a 5- or 6-membered heterocyclic group containing at least one ring heteroatom selected from nitrogen, oxygen and sulphur, or R³ and R⁴ together form a 5- or 6-membered heterocyclic group containing at least one ring heteroatom selected from nitrogen, oxygen and sulphur, and R⁵, R⁶ and R⁷ are independently hydrogen or C₁-C₈-alkyl.

2. A compound according to claim 1, where
R¹ is hydrogen;
R² is phenyl substituted at one position by fluoro and at another position by fluoro, chloro or C₁-C₈-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂, C₁-C₈-alkylthio optionally substituted by C₆-C₁₀-aryl, C₁-C₈-alkylsulfonyl optionally substituted by C₆-C₁₀-aryl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is a 5- or 6-membered heterocyclic group containing at least one ring heteroatom selected from nitrogen, oxygen and sulphur, and R³ and R⁴ are both hydrogen;
or R² is C₁-C₈-alkyl optionally substituted by a 5- or 6-membered heterocyclic group containing at least one ring heteroatom selected from nitrogen, oxygen and sulphur, and R³ and R⁴ are independently hydrogen or halo.

3. A compound according to claim 1, where
R¹ is hydrogen;
R² is phenyl substituted at one position by fluoro and at another position by fluoro, chloro or C₁-C₄-alkyl, and R³ and R⁴ are both hydrogen;
or R² is phenyl monosubstituted by chloro or phenyl, and R³ and R⁴ are both hydrogen;
or R² is phenyl substituted at one, two or three positions by halo, and one of R³ and R⁴ is hydrogen, and the other of R³ and R⁴ is -SO₂-NH₂, C₁-C₄-alkylthio optionally substituted by phenyl, C₁-C₄-alkylsulfonyl optionally substituted by phenyl, or a 5- or 6-membered heterocyclic group containing at least one ring heteroatom selected from nitrogen, oxygen and sulphur;
or R² is phenyl substituted at one, two or three positions by halo, R³ is hydrogen, and R⁴ is fluoro;
or R² is a 5- or 6-membered heterocyclic group containing at least one ring heteroatom selected from nitrogen, oxygen and sulphur, and R³ and R⁴ are both hydrogen;
or R² is C₁-C₄-alkyl optionally substituted by a 5- or 6-membered heterocyclic group containing at least one ring heteroatom selected from nitrogen, oxygen and sulphur, and R³ and R⁴ are independently hydrogen or halo.

4. A compound according to claim 1 as defined in the following table:

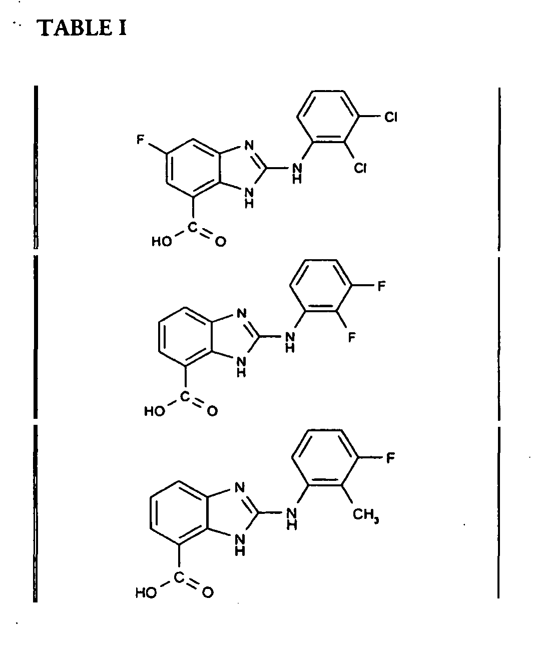

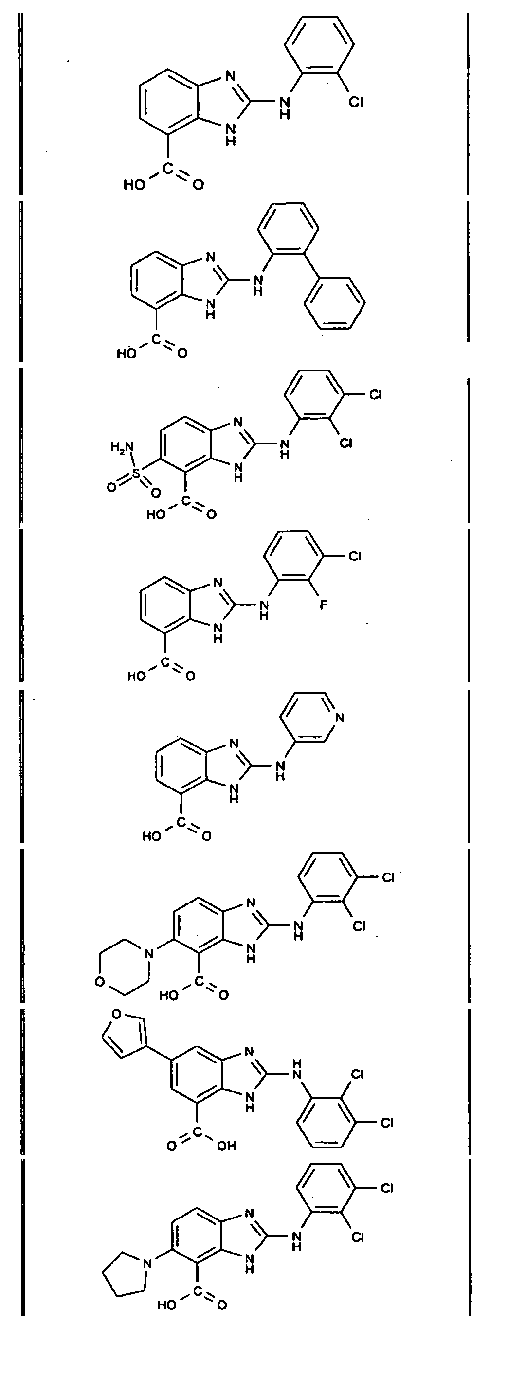

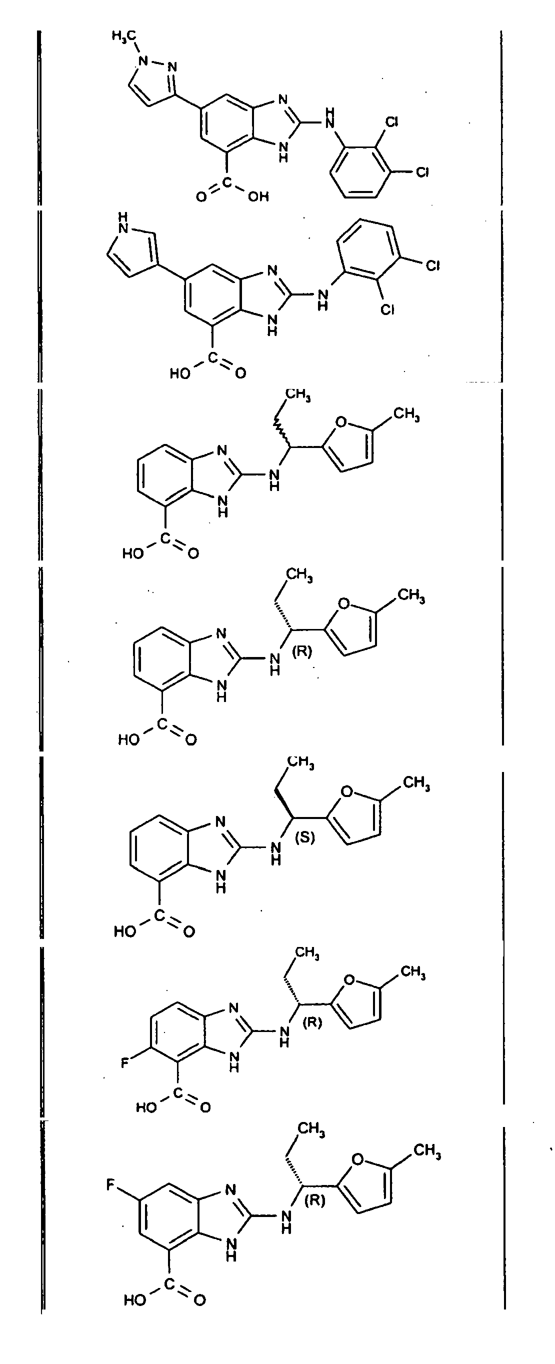

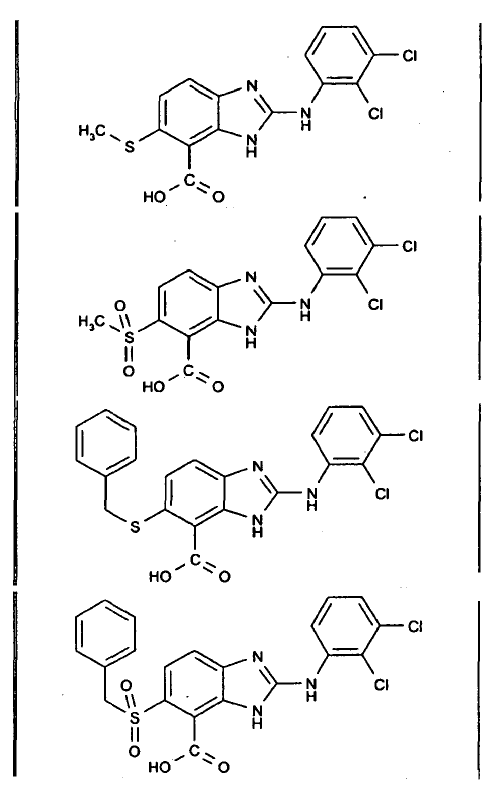

5. A compound according to any one of the preceding claims for use as a pharmaceutical.

6. A compound according to any one of claims 1 to 4 in combination with an anti-inflammatory, bronchodilatory, antihistamine or anti-tussive drug substance, said compound and said drug substance being in the same or different pharmaceutical composition.

7. A pharmaceutical composition comprising as active ingredient a compound according to any one of claims 1 to 4, optionally together with a pharmaceutically acceptable diluent or carrier.

8. The use of a compound according to any one of claims 1 to 4 for the manufacture of a medicament for the treatment of a condition mediated by the CXCR2 receptor.

9. The use of a compound according to any one of claims 1 to 4 for the manufacture of a medicament for the treatment of an inflammatory or allergic condition, particularly an inflammatory or obstructive airways disease.

10. A process for the preparation of a compound of formula I as claimed in claim 1 which comprises
(i) (A) for the preparation of compounds of formula I, cyclising a compound of formula IIa or IIb in either case optionally in protected form 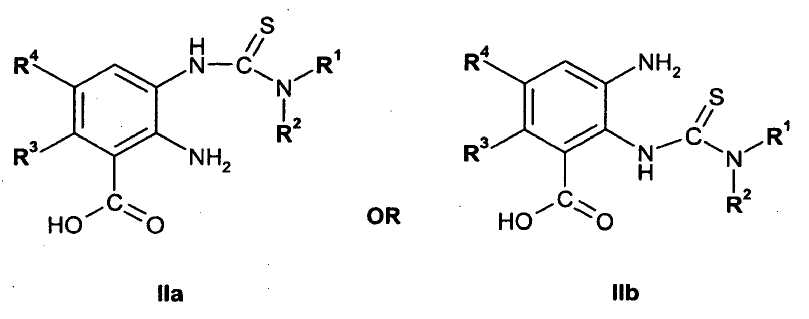 where R¹, R², R³, and R⁴ are as defined in claim 1, and deprotecting where necessary;
(B) for the preparation of compounds of formula I where R¹ is hydrogen, reacting a compound of formula IIa or IIIb in either case optionally in protected form 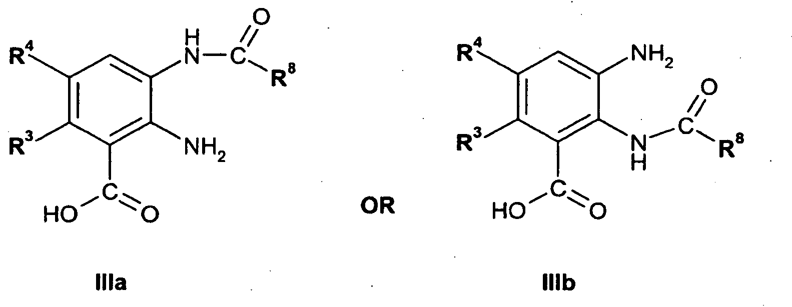 where R³ and R⁴ are as defined in claim 1 and R⁸ is C₁-C₄-alkyl, with a compound of formula IV
S=C=N-R² **IV**
where R² is as defined in claim 1in the presence of a coupling agent, and deprotecting where necessary; or
(C) for the preparation of compounds of formula I where at least one of R³ and R⁴ is C₁-C₈-alkylsulfinyl or C₁-C₈-alkylsulfonyl in either case optionally substituted by C₆-C₁₀-aryl, oxidising a compound of formula I where R¹ and R² are as defined in claim 1, and at least one of R³ and R⁴ as defined in claim 1 is C₁-C₈-alkylthio optionally substituted by C₆-C₁₀-aryl, or a protected form thereof, and deprotecting where necessary; and
(ii) recovering the product in free or salt form.

## Patentansprüche

1. Verbindung der Formel I 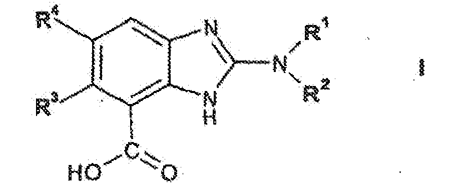 in freier Form oder Salzform, wobei
R¹ für Wasserstoff oder C₁-C₈, Alkyl steht;
R² für Phenyl, das an einer Position durch Fluor und an einer anderen Position durch Fluor, Chlor oder C₁-C₈-Alkyl substituiert ist, steht und R³ und R⁴ beide für Wasserstoff stehen;
oder R² für Phenyl, das einfach durch Chlor oder Phenyl substituiert ist, steht und R³ und R⁴ beide für Wasserstoff stehen;
oder R² für Phenyl, das an einer, zwei oder drei Positionen durch Halogen substituiert ist, steht und eine der Gruppen R³ und R⁴ für Wasserstoff steht und die andere für -SO₂-NH₂, C₁-C₈-Alkylthio, das gegebenenfalls durch C₆-C₁₀-Aryl substituiert ist, C₁-C₈-Alkylsulfinyl, das gegebenenfalls durch C₆-C₁₀-Aryl substituiert ist, C₁-C₈-Alkylsulfonyl, das gegebenenfalls durch C₆-C₁₀-Aryl substituiert ist, oder eine 5- oder 6-gliedrige heterocyclische Gruppe mit mindestens einem unter Stickstoff, Sauerstoff und Schwefel ausgewählten Ringheteroatom steht;
oder R² für Phenyl, das an einer, zwei oder drei Positionen durch Halogen substituiert ist, steht, R³ für Wasserstoff steht und R⁴ für Fluor steht;
oder R² für eine 5- oder 6-gliedrige heterocyclische Gruppe mit mindestens einem unter Stickstoff, Sauerstoff und Schwefel ausgewählten Ringheteroatom steht, R³ und R⁴ unabhängig voneinander für Wasserstoff, Halogen, Cyano, Hydroxy, Nitro, Carboxy, Aminocarbonyl, C₁-C₈-Alkyl, C₁-C₈-Alkenyl, C₁-C₈-Alkinyl, C₁-C₈-Halogenalkyl, C₁-C₈-Alkylthio, das gegebenenfalls durch C₆-C₁₀-Aryl substituiert ist, C₁-C₈-Alkylsulfinyl, das gegebenenfalls durch C₆-C₁₀-Aryl substituiert ist, C₁-C₈-Alkylsulfonyl, das gegebenenfalls durch C₆-C₁₀-Aryl substituiert ist, C₁-C₈-Alkoxy, C₁-C₈-Halogenalkoxy, C₃-C₈-Cycloalkyl, C₃-C₈-Cycloalkyl-C₁-C₈-alkyl, C₃-C₈-Cycloalkyloxy, -CO-NR⁵R⁶, -NH-SO₂R⁷ -NH-COH, -SO₂NH₂ oder eine 5- oder 6-gliedrige heterocyclische Gruppe mit mindestens einem unter Stickstoff, Sauerstoff und Schwefel ausgewählten Ringheteroatom stehen oder R³ und R⁴ zusammen eine 5- oder 6-gliedrige heterocyclische Gruppe mit mindestens einem unter Stickstoff, Sauerstoff und Schwefel ausgewählten Ringheteroatom bilden und R⁵, R⁶ und R⁷ unabhängig voneinander für Wasserstoff oder C₁-C₈-Alkyl stehen;
oder R² für C₁-C₈-Alkyl, das gegebenenfalls durch eine 5- oder 6-gliedrige heterocyclische Gruppe mit mindestens einem unter Stickstoff, Sauerstoff und Schwefel ausgewählten Ringheteroatom substituiert ist, steht, R³ und R⁴ unabhängig voneinander für Wasserstoff, Halogen, Cyano, Hydroxy, Nitro, Carboxy, Aminocarbonyl, C₁-C₈-Alkyl, C₁-C₈-Alkenyl, C₁-C₈-Alkinyl, C₁-C₈-Halogenalkyl, C₁-C₈-Alkylthio, das gegebenenfalls durch C₆-C₁₀-Aryl substituiert ist, C₁-C₈-Alkylsulfinyl, das gegebenenfalls durch C₆-C₁₀-Aryl substituiert ist, C₁-C₈-Alkylsulfonyl, das gegebenenfalls durch C₆-C₁₀-Aryl substituiert ist, C₁-C₈-Alkoxy, C₁-C₈-Halogenalkoxy, C₃-C₈-Cycloalkyl, C₃-C₈-Cycloalkyl-C₁-C₈-alkyl, C₃-C₈-Cycloalkyloxy, -CO-NR⁵R⁶, -NH-SO₂R⁷, -NH-COH, -SO₂NH₂ oder eine 5- oder 6-gliedrige heterocyclische Gruppe mit mindestens einem unter Stickstoff, Sauerstoff und Schwefel ausgewählten Ringheteroatom stehen oder R³ und R⁴ zusammen eine 5- oder 6-gliedrige heterocyclische Gruppe mit mindestens einem unter Stickstoff, Sauerstoff und Schwefel ausgewählten Ringheteroatom bilden und R⁵, R⁶ und R⁷ unabhängig voneinander für Wasserstoff oder C₁-C₈-Alkyl stehen.

2. Verbindung nach Anspruch 1, wobei
R¹ für Wasserstoff steht;
R² für Phenyl, das an einer Position durch Fluor und an einer anderen Position durch Fluor, Chlor oder C₁-C₈-alkyl substituiert ist, steht und R³ und R⁴ beide für Wasserstoff stehen;
oder R² für Phenyl, das einfach durch Chlor oder Phenyl substituiert ist, steht und R³ und R⁴ beide für Wasserstoff stehen;
oder R² für Phenyl, das an einer, zwei oder drei Positionen durch Halogen substituiert ist, steht und eine der Gruppen R³ und R⁴ für Wasserstoff steht und die andere für -SO₂-NH₂, C₁-C₈-Alkylthio, das gegebenenfalls durch C₆-C₁₀-Aryl substituiert ist, C₁-C₈-Alkylsulfonyl, das gegebenenfalls durch C₆-C₁₀-Aryl substituiert ist, oder eine 5- oder 6-gliedrige heterocyclische Gruppe mit mindestens einem unter Stickstoff, Sauerstoff und Schwefel ausgewählten Ringheteroatom steht;
oder R² für Phenyl, das an einer, zwei oder drei Positionen durch Halogen substituiert ist, steht, R³ für Wasserstoff steht und R⁴ für Fluor steht;
oder R² für eine 5- oder 6-gliedrige heterocyclische Gruppe mit mindestens einem unter Stickstoff, Sauerstoff und Schwefel ausgewählten Ringheteroatom steht und R³ und R⁴ beide für Wasserstoff stehen;
oder R³ für C₁-C₈-Alkyl, das gegebenenfalls durch eine 5- oder 6-gliedrige heterocyclische Gruppe mit mindestens einem unter Stickstoff, Sauerstoff und Schwefel ausgewählten Ringheteroatom steht und R³ und R⁴ unabhängig voneinander für Wasserstoff oder Halogen stehen.

3. Verbindung nach Anspruch 1, wobei
R¹ für Wasserstoff steht;
R² für Phenyl, das an einer Position durch Fluor und an einer anderen Position durch Fluor, Chlor oder C₁-C₄-Alkyl substituiert ist, steht und R³ und R⁴ beide für Wasserstoff stehen;
oder R² für Phenyl, das einfach durch Chlor oder Phenyl substituiert ist, steht und R³ und R⁴ beide für Wasserstoff stehen;
oder R² für Phenyl, das an einer, zwei oder drei Positionen durch Halogen substituiert ist, steht und eine der Gruppen R³ und R⁴ für Wasserstoff steht und die andere für -SO₂-NH₂, C₁-C₄-Alkylthio, das gegebenenfalls durch Phenyl substituiert ist, C₁-C₄-Alkylsulfonyl, das gegebenenfalls durch Phenyl substituiert ist, oder eine 5- oder 6-gliedrige heterocyclische Gruppe mit mindestens einem unter Stickstoff, Sauerstoff und Schwefel ausgewählten Ringheteroatom steht;
oder R² für Phenyl, das an einer, zwei oder drei Positionen durch Halogen substituiert ist, steht, R³ für Wasserstoff steht und R⁴ für Fluor steht;
oder R² für eine 5- oder 6-gliedrige heterocyclische Gruppe mit mindestens einem unter Stickstoff, Sauerstoff und Schwefel ausgewählten Ringheteroatom steht und R³ und R⁴ beide für Wasserstoff stehen;
oder R² für C₁-C₄-Alkyl, das gegebenenfalls durch eine 5- oder 6-gliedrige heterocyclische Gruppe mit mindestens einem unter Stickstoff, Sauerstoff und Schwefel ausgewählten Ringheteroatom substituiert ist, steht und R³ und R⁴ unabhängig voneinander für Wasserstoff oder Halogen stehen.

4. Verbindung nach Anspruch 1 gemäß der folgenden Tabelle:

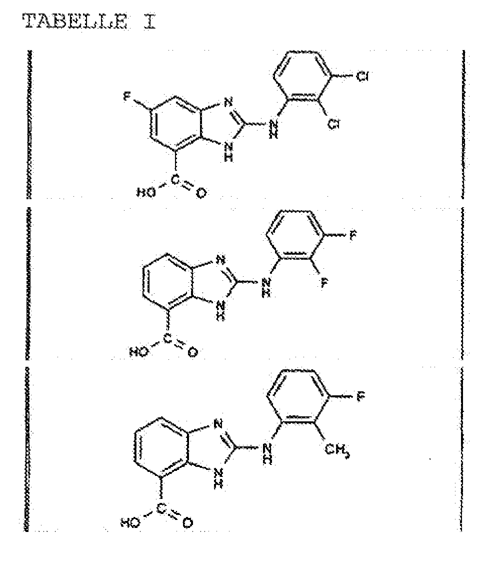

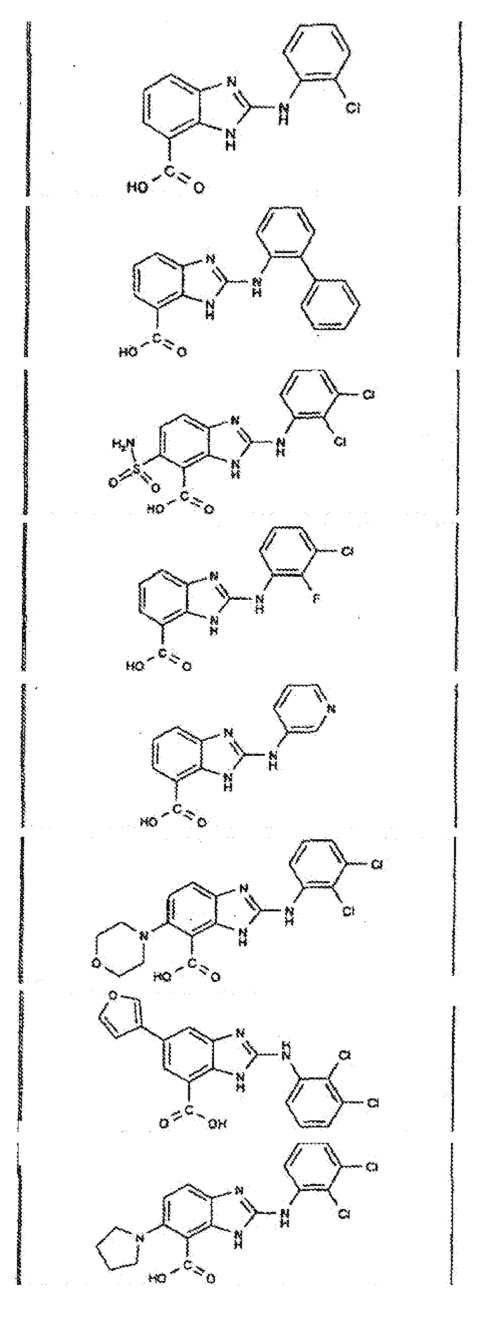

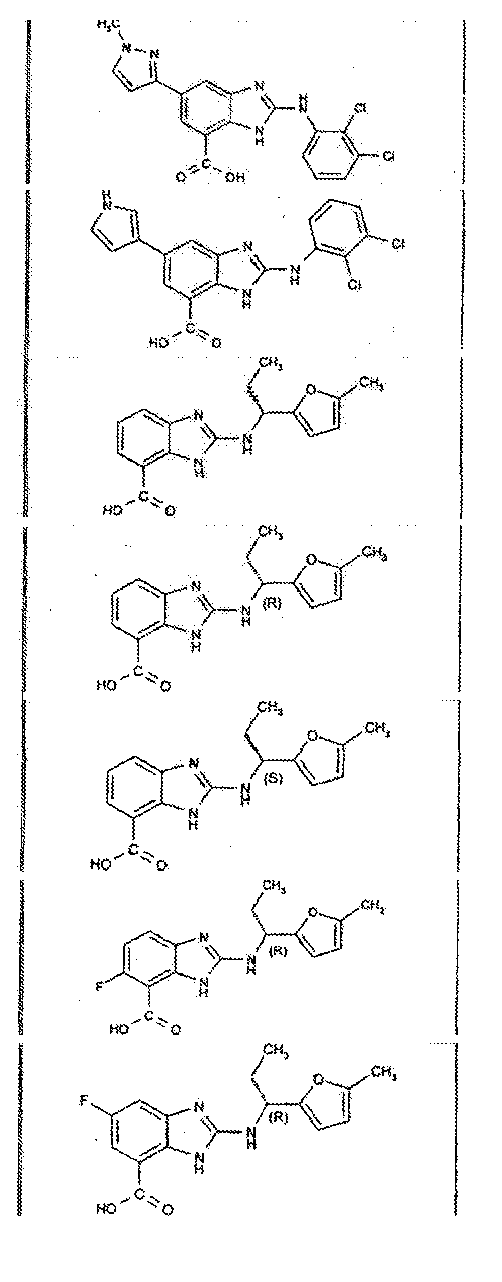

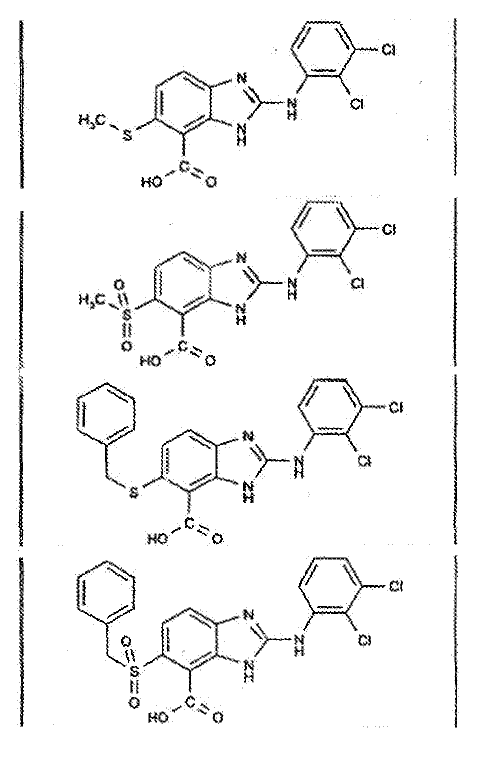

5. Verbindung nach einem der vorhergehenden Ansprüche zur Verwendung als Pharmazeutikum.

6. Verbindung nach einem der Ansprüche 1 bis 4 in Kombination mit einem entzündungshemmenden oder bronchodilatorischen Arzneistoff oder einem Arzneistoff mit Antihistaminwirkung oder antitussiver Wirkung, wobei die Verbindung und der Arzneistoff in der gleichen pharmazeutischen Zusammensetzung oder in verschiedenen pharmazeutischen Zusammensetzungen vorliegen.

7. Pharmazeutische Zusammensetzung, die als Wirkstoff eine Verbindung nach einem der Ansprüche 1 bis 4 umfasst, gegebenenfalls zusammen mit einem pharmazeutisch unbedenklichen Verdünnungsmittel oder Träger.

8. Verwendung einer Verbindung nach einem der Ansprüche 1 bis 4 zur Herstellung eines Arzneimittels zur Behandlung eines durch den CXCR2-Rezeptor vermittelten Leidens,

9. Verwendung einer Verbindung nach einem der Ansprüche 1 bis 4 zur Herstellung eines Arzneimittels zur Behandlung eines entzündlichen oder allergischen Leidens, insbesondere einer entzündlichen oder obstruktiven Atemwegserkrankung.

10. Verfahren zur Herstellung einer Verbindung der Formel I gemäß Anspruch 1, bei dem man
(i) (A) zur Herstellung von Verbindungen der Formel I, eine Verbindung der Formel IIa oder IIb jeweils gegebenenfalls in geschützter Form 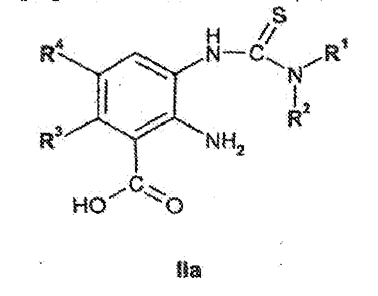 oder 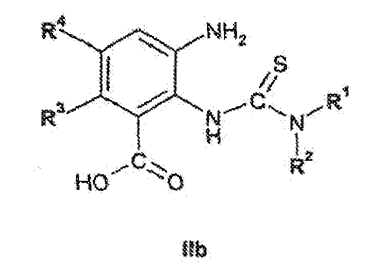 wobei R¹, R², R³ und R⁴ die in Anspruch 1 angegebene Bedeutung besitzen, cyclisiert und gegebenenfalls entschützt;
(B) zur Herstellung von Verbindungen der Formel I, wobei R¹ für Wasserstoff steht, eine Verbindung der Formel IIa oder IIIb jeweils gegebenenfalls in geschützter Form 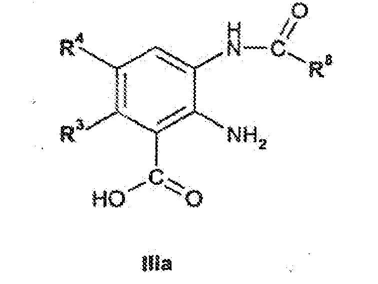 oder 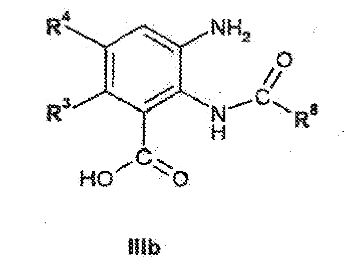 wobei R³ und R⁴ die in Anspruch 1 angegebene Bedeutung besitzen und R⁸ für C₁-C₄-Alkyl steht, in Gegenwart eines Kupplungsmittels mit einer Verbindung der Formel IV
S = C = N-R² **IV**
worin R² die in Anspruch 1 angegebene Bedeutung beisitzt, umsetzt und gegebenenfalls entschutzt; oder
(C) zur Herstellung von Verbindungen der Formel I, wobei mindestens eine der Gruppen R³ und R⁴ für C₁-C₈-Alkylsulfinyl oder C₁-C₈-Alkylsulfonyl, jeweils gegebenenfalls durch C₆-C₁₀-Aryl substituiert, steht, eine Verbindung der Formel I, wobei R¹ und R² die in Anspruch 1 angegebene Bedeutung besitzen und mindestens eine der Gruppen R³ und R⁴ gemäß der in Anspruch 1 angegebenen Definition für C₁-C₈-Alkylthio, das gegebenenfalls durch C₆-C₁₀-Aryl substituiert ist, oder eine geschützte Form davon steht, oxidiert und gegebenenfalls entschützt; und
(ii) das Produkt in freier Form oder in Salzform gewinnt.

## Revendications

1. Composé de formule I 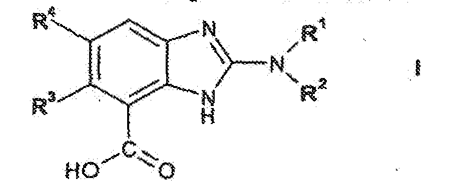 sous forme libre ou sous forme de sel, où
R¹ représente un atome d'hydrogène ou un groupement alkyle en C₁-C₈ ;
R² représente un groupement phényle substitué en une position par un groupement fluoro et en une autre position par un groupement fluoro, chloro ou alkyle en C₁-C₈, et R³ et R⁴ représentent tous deux des atomes d'hydrogène ;
ou R² représente un groupement phényle monosubstitué par un groupement chloro ou un groupement phényle, et R³ et R⁴ représentent tous deux des atomes d'hydrogène ;
ou R² représente un groupement phényle substitué en une, deux ou trois positions par des groupements halogène, et l'un des groupements R³ et R⁴ représente un atome d'hydrogène, et l'autre groupement R³ ou R⁴ représente un groupement -SO₂-NH₂, (alkyle en C₁-C₃)-thio éventuellement substitué par un groupement aryle en C₆-C₁₀, (alkyle en C₁-C₈)-sulfinyle éventuellement substitué par un groupement aryle en C₆-C₁₀, (alkyle en C₁-C₈)-sulfonyle éventuellement substitué par un groupement aryle en C₆-C₁₀, ou un groupement hétérocyclique à 5 ou 6 chaînons contenant au moins un hétéroatome de cycle choisi parmi l'azote, l'oxygène et le soufre ;
ou R² représente un groupement phényle substitué en une, deux ou trois positions par des groupements halogène, R³ représente un atome d'hydrogène et R⁴ représente un groupement fluoro ;
ou R² représente un groupement hétérocyclique à 5 on 6 chaînons comportant au moins un hétèroatome de cycle choisi parmi l'azote, l'oxygène et le soufre, R³ et R⁴ représentent indépendamment un atome d'hydrogène ou un groupement halogéno, cyano, hydroxy, nitro, carboxy, aminocarbonyle, alkyle en C₁-C₈, alcényle en C₁-C₈, alcynyle en C₁-C₈, halogénoalkyle en C₁-C₈, (alkyle en C₁-C₈)-thio éventuellement substitué par un groupement aryle en C₆-C₁₀, (alkyle en C₁-C₈)-sulfinyle éventuellement substitué par un groupement aryle en C₆-C₁₀, (alkyle en C₁-C₈)-sulfonyle éventuellement substitué par un groupement aryle en C₆-C₁₀, alkoxy en C₁-C₈, halogénoalkoxy en C₁-C₈, cycloalkyle en C₃-C₈, (cycloalkyle en C₃-C₈)-(alkyle en C₁-C₈), cycloalkyloxy en C₃-C₈, -CO-NR⁵R⁶, -NH-SO₂R⁷, -NH-COH, -SO₂NH₂ ou un groupement hétérocyclique à 5 ou 6 chaînons comportant au moins un hétéroatome de cycle choisi parmi l'azote, l'oxygène ou le soufre, ou R³ et R⁴ forment ensemble un groupement hétérocyclique à 5 ou 6 chaînons comportant au moins un hétéroatome de cycle choisi parmi l'azote, l'oxygène et le soufre, et R⁵, R⁶ et R⁷ représentent indépendamment un atome d'hydrogène ou un groupement alkyle en C₁-C₈ ; ou
ou R² représente un groupement alkyle en C₁-C₈ éventuellement substitué par un groupement hétérocyclique à 5 ou 6 chaînons comportant au moins un hétéroatome de cycle choisi parmi l'azote, l'oxygène et le soufre, R³ et R⁴ représentent indépendamment un atome d'hydrogène ou un groupement halogéno, cyano, hydroxy, nitro, carboxy, aminocarbonyle, alkyle en C₁-C₈, alcényle en C₁-C₈, alcynyle en C₁-C₈, halogénoalkyle en C₁-C₈, (alkyle en C₁-C₈)-thio éventuellement substitué par un groupement aryle en C₆-C₁₀, (alkyle en C₁-C₈)-sulfinyle éventuellement substitué par un groupement aryle en C₆-C₁₀, (alkyle en C₁-C₈)-sulfonyle éventuellement substitué par un groupement aryle en C₆-C₁₀, alkoxy en C₁-C₈, halogénoalkoxy en C₁-C₈, cycloalkyle en C₃-C₈, (cycloalkyle en C₃-C₈)-(alkyle en C₁-C₈), cycloalkyloxy en C₃-C₈, -CO-NR⁵R⁶, -NH-SO₂R⁷, -NH-COH, -SO₂NH₂ ou un groupement hétérocyclique à 5 ou 6 chaînons comportant au moins un hétéroatome de cycle choisi parmi l'azote, l'oxygène et le soufre, ou R³ et R⁴ forment ensemble un groupement hétérocyclique à 5 ou 6 chaînons comportant au moins un hétéroatome de cycle choisi parmi l'azote, l'oxygène et le soufre, et R⁵, R⁶ et R⁷ représentent indépendamment un atome d'hydrogène ou un groupement alkyle en C₁-C₈.

2. Composé conforme à la revendication 1, où
R¹ représente un atome d'hydrogène ;
R² représente un groupement phényle substitué en une position par un groupement fluoro et en une autre position par un groupement fluoro, chloro ou alkyle en C₁-C₈, et R³ et R⁴ représentent tous deux des atomes d'hydrogène ;
ou R² représente un groupement phényle monosubstitué par un groupement chloro ou un groupement phényle, et R³ et R⁴ représentent tous deux des atomes d'hydrogène ;
ou R² représente un groupement phényle substitué en une, deux ou trois positions par des groupements halogéno, et l'un des groupements R³ et R⁴ représente un atome d'hydrogène, et l'autre groupement R³ ou R⁴ représente un groupement -SO₂-NH₂, (alkyl, en C₁-C₈)-thio éventuellement substitué par un groupement aryle en C₆-C₁₀, (alkyle en C₁-C₈)-sulfonyle éventuellement substitué par un groupement aryle en C₆-C₁₀, ou un groupement hétérocyclique à 5 ou 6 chaînons comportant au moins un hétéroatome de cycle choisi parmi l'azote, l'oxygène et le soufre :
ou R² représente un groupement phényle substitué en une, deux ou trois positions par des groupements halogéno, R³ représente un atome d'hydrogène et R⁴ représente un groupement fluoro ;
ou R² représente un groupement hétérocyclique à 5 ou 6 chaînons comportant au moins un hétéroatome de cycle choisi parmi l'azote, l'oxygène et le soufre, et R³ et R⁴ représentent tous deux des atomes d'hydrogène ;
ou R² représente un groupement alkyle en C₁-C₈ éventuellement substitué par une groupement hétérocyclique à 5 ou 6 chaînons comportant au moins un hétéroatome de cycle choisi parmi l'azote, l'oxygène et le soufre, et R³ et R⁴ représentent indépendamment un atome d'hydrogène ou un groupement halogéno.

3. Composé conforme à la revendication 1, où
R¹ représente un atome d'hydrogène ;
R² représente un groupement phényle substitué en une position par un groupement fluoro et en une autre position par un groupement fluoro, chloro ou alkyle en C₁-C₄, et R³ et R⁴ représentent tous deux des atomes d'hydrogène ;
ou R² représente un groupement phényle monosubstitué par un groupement chloro ou un groupement phényle, et R³ et R⁴ représentent tous deux des atomes d'hydrogène ;
ou R² représente un groupement phényle substitué en une, deux ou trois positions par des groupements halogéno, et l'un des groupements R³ et R⁴ représente un atome d'hydrogène, et l'autre groupement R³ ou R⁴ représente un groupement -SO₂-NH₂, (alkyle en C₁-C₄)-thio éventuellement substitué par un groupement phényle, (alkyle en C₁-C₄)-sulfonyle éventuellement substitué par un groupement phényle ou un groupement hétérocyclique à 5 ou 6 chaînons comportant au moins un hétéroatome de cycle choisi parmi l'azote, l'oxygène et le soufre ;
ou R² représente un groupement phényle substitué en une, deux ou trois positions par des groupements halogéno, R³ représente un atome d'hydrogène et R⁴ représente un groupement fluoro ;
ou R² représente un groupement hétérocyclique à 5 ou 6 chaînons comportant au moins un hétéroatome de cycle choisi parmi l'azote, l'oxygène et le soufre, et R³ et R⁴ représentent tous deux des atomes d'hydrogène ;
ou R² représente un groupement alkyle en C₁-C₄ éventuellement substitué par un groupement hétérocyclique à 5 ou 6 chaînons comportant au moins un hétéroatome de cycle choisi parmi l'azote, l'oxygène et le soufre, et R³ et R⁴ représentent indépendamment un atome d'hydrogène ou un groupement halogéno,

4. composé conforme à la revendication 1 comme défini dans le tableau suivant :

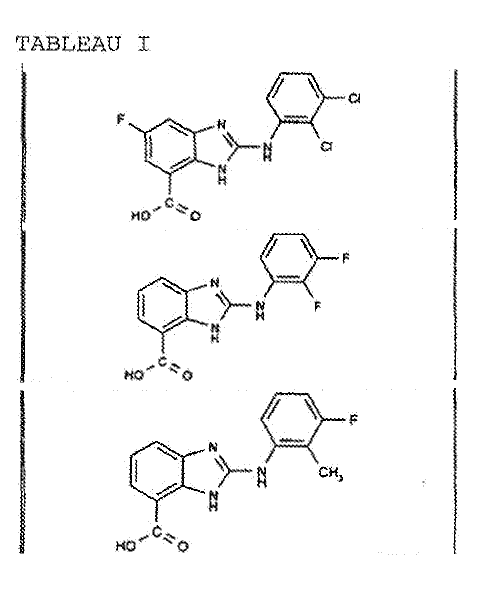

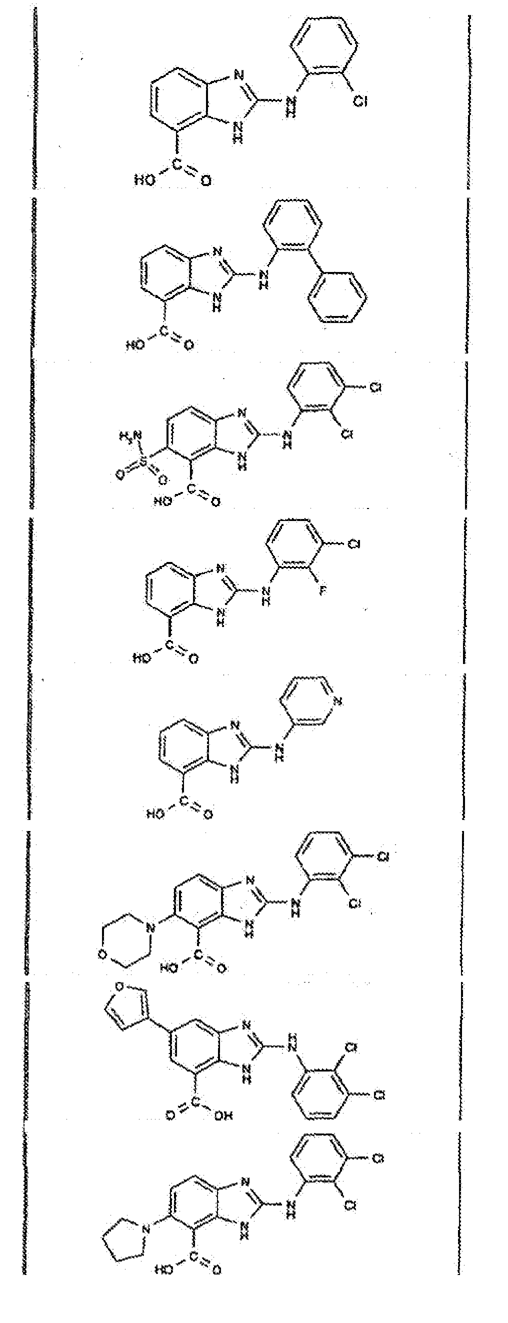

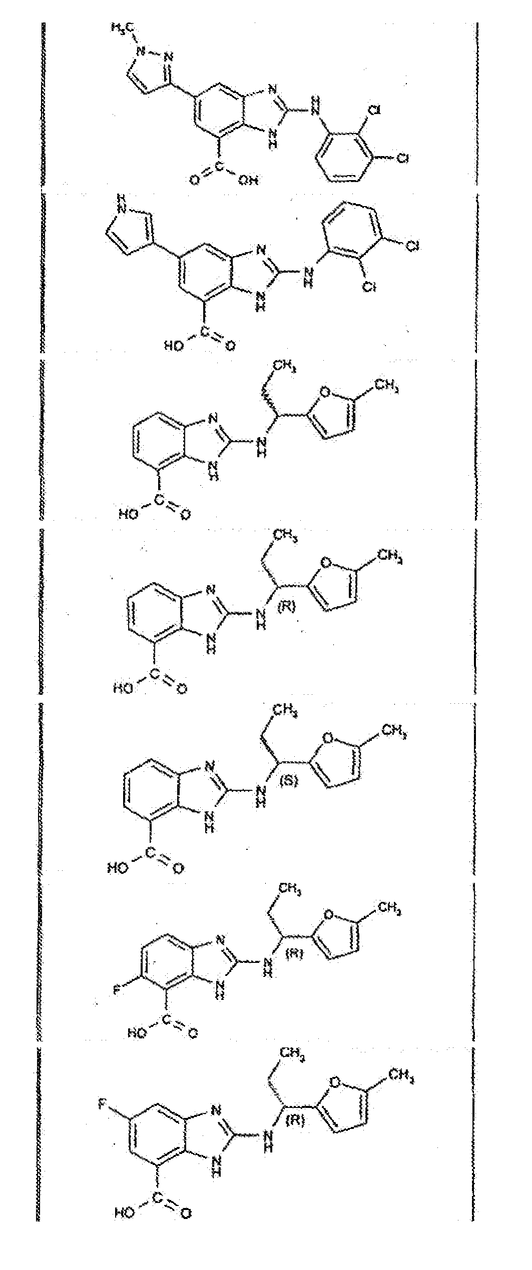

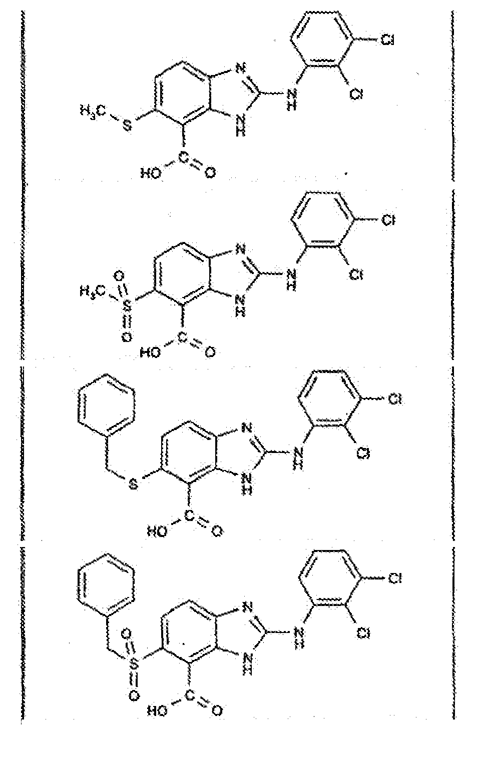

5. Composé conforme à l'une quelconque des revendications précédentes pour emploi en tant que produit pharmaceutique.

6. Composé conforme à l'une quelconque des revendications 1 à 4 combiné à une substance médicamenteuse anti-inflammatoire, bronchodilatatrice, antihistaminique ou antitussive, ledit composé et ladite substance médicamenteuse étant compris dans la même composition pharmaceutique ou dans des compositions pharmaceutiques différentes.

7. Composition pharmaceutique comprenant, au titre de principe actif, un composé conforme à l'une quelconque des revendications 1 à 4, éventuellement associé à un diluant ou vecteur de qualité pharmaceutique.

8. Emploi d'un composé conforme à l'une quelconque des revendications 1 à 4 dans la fabrication d'un médicament destiné au traitement d'un état pathologique faisant intervenir le récepteur CXCR2.

9. Emploi d'un composé conforme à l'une quelconque des revendications 1 à 4 dans la fabrication d'un médicament destiné au traitement d'un état inflammatoire ou allergique, particulièrement d'une pathologie inflammatoire ou obstructive des voies respiratoires.

10. Procédé de synthèse d'un compose de formule I conforme à la revendication 1, comprenant
(i) (A) pour la synthèse des composés de formule I, la cyclisation d'un composé de formule IIa ou IIb, dans chaque cas, éventuellement, sous forme protégée 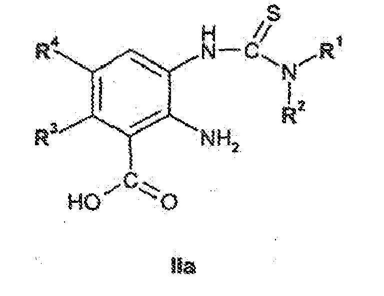 où 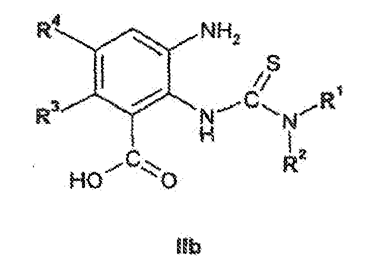 où R¹, R², R³ et R⁴ sont tels que définis dans la revendication 1, et la déprotection le cas échéant ;
(B) pour la synthèse des composés de formule I où R¹ représente un atome d'hydrogène, la réaction d'un composé de formule IIIa ou IIIb, dans chaque cas, éventuellement, sous forme protégée 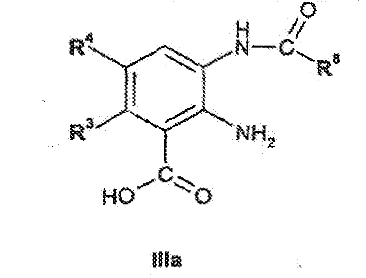 où 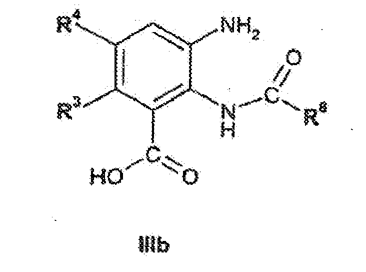 où R³ et R⁴ sont tels que définis dans la revendication 1 et R⁸ représente un groupement alkyle en C₁-C₄, avec un composé de formule IV
S = C = N-R² IV
où R² est tel que défini dans la revendication 1 en présence d'un agent de couplage, et la déprotection le cas échéant ; ou
(C) pour la synthèse de composés de formule I où au moins l'un des radicaux R³ et R⁴ représente un groupement (alkyle en C₁-C₈)-sulfinyle ou (alkyle en C₁-C₈)-sulfonyle, dans chaque cas éventuellement substitué par un groupement aryle en C₆-C₁₀, l'oxydation d'un composé de formule I où R¹ et R² sont tels que définis dans la revendication 1, et au moins l'un des radicaux R³ et R⁴ tels que définis dans la revendication 1 représente un groupement (alkyle en C₁-C₈)-thio éventuellement substitué par un groupement aryle en C₆-C₁₀, ou l'une de ses formes protégée, et la déprotection le cas échéant : et
(ii) la récupération du produit sous forme libre ou sous forme de sel.
